(19) Europäisches Patentamt
European Patent Office
Office européen des brevets

(11) **EP 3 399 914 B1**

(12) **EUROPÄISCHE PATENTSCHRIFT**

(45) Veröffentlichungstag und Bekanntmachung des Hinweises auf die Patenterteilung:
**30.10.2019 Patentblatt 2019/44**

(21) Anmeldenummer: **17700502.2**

(22) Anmeldetag: **06.01.2017**

(51) Int Cl.:
***A61B 5/1455*** (2006.01)

(86) Internationale Anmeldenummer:
**PCT/EP2017/050272**

(87) Internationale Veröffentlichungsnummer:
**WO 2017/118735 (13.07.2017 Gazette 2017/28)**

(54) **VORRICHTUNG UND VERFAHREN ZUR KONTINUIERLICHEN UND NICHT INVASIVEN BESTIMMUNG VON PHYSIOLOGISCHEN PARAMETERN EINES PROBANDEN**

DEVICE AND METHOD FOR THE CONTINUOUS AND NON-INVASIVE DETERMINATION OF PHYSIOLOGICAL PARAMETERS OF A TEST SUBJECT

DISPOSITIF ET PROCÉDÉ DE DÉTERMINATION CONTINUE ET NON-INVASIVE DE PARAMÈTRES PHYSIOLOGIQUES D'UN SUJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.01.2016 EP 16150553**

(43) Veröffentlichungstag der Anmeldung:
**14.11.2018 Patentblatt 2018/46**

(73) Patentinhaber: **Oxy4 GmbH**
**18233 Am Salzhaff/OT Pepelow (DE)**

(72) Erfinder:
• **KULCKE, Axel**
**18233 Am Salzhaff/ OT Pepelow (DE)**

• **HOLMER, Amadeus**
**18209 Bad Doberan (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**WO-A1-2014/139830     US-A1- 2009 326 347**
**US-A1- 2012 253 153**

EP 3 399 914 B1

**Beschreibung**

Die vorliegende Erfindung betrifft eine Vorrichtung zur kontinuierlichen und nichtinvasiven Bestimmung von physiologischen Parametern eines Probanden bei körperlicher Belastung mit:

einer zur Auflage auf die Haut des Probanden an einer gewünschten Messstelle ausgestaltete Beleuchtungseinheit auf LED-Basis mit einer Mehrzahl von unterschiedlichen, nebeneinander angeordneten LED-Typen, deren Emissionsmaxima bei verschiedenen Wellenlängen $\lambda 1$, $\lambda 2$ ... $\lambda L$ vom sichtbaren bis in den NIR-Wellenlängenbereich liegen,

einem Fotosensor, der zur Auflage auf die Haut des Probanden ausgestaltet ist, um von der Beleuchtungseinheit ausgesendetes und durch den Körper des Probanden zu einem Austrittsort im Bereich des Fotosensors gelangendes Licht zu erfassen, und

einer Datenverarbeitungseinheit, die zum Auslesen des Fotosensors mit diesem verbunden ist und die mit der Beleuchtungseinheit in Verbindung steht und dazu eingerichtet ist, die unterschiedlichen LED-Typen jeweils einzeln in einer vorgegebenen Aktivierungssequenz zu aufeinanderfolgenden Aktivierungsstartzeitpunkten $t_k$ $(k=1, 2 ... M)$ für eine jeweils vorgegebene Aktivierungsdauer zu aktivieren und die Aktivierungssequenz mit einer Taktfrequenz als Folge $n= 1, 2 ... N$ von Aktivierungssequenzen zu wiederholen, wobei die Taktfrequenz ausreichend hoch zur Auflösung des Pulses des Kreislaufs des Probanden ist.

Die Beurteilung des Trainingszustands eines Sportlers, die Messbarkeit seines Trainingsfortschrittes und die Aufstellung eines möglichst effektiven und effizienten Trainingsplanes ist eine anspruchsvolle Aufgabe, deren Relevanz auch wirtschaftlich immer mehr in den Mittelpunkt der Betrachtungsweise des modernen Sports rückt. Eine umfassende Datenerfassung über den Grad der Beanspruchung der aktiven Muskulatur unter definierten Leistungsanforderungen und im Training ist die Voraussetzung zum Erhalt objektiver Beurteilungskriterien, die einerseits zur Sicherung der körperlichen Unversehrtheit als auch zur Leistungsoptimierung unerlässlich sind. Dabei wäre es wünschenswert, Informationen sowohl über die Atmung (Lunge), den Bluttransport (Herzkreislauf) als auch die Muskulatur (Energieumsatz) zu liefern.

Zur sportphysiologischen Leistungsbeurteilung werden heutzutage vorwiegend die Verfahren der Spiroergometrie und der Laktatdiagnostik eingesetzt. Weiterhin kommen ergänzend häufig Pulsoximeter aus dem medizinischen Bereich zum Einsatz, um die Pulsrate und die arterielle Sauerstoffsättigung ($SpO_2$) zu bestimmen. Bei der Grundtechnologie der Pulsoximetrie ist es üblich, zwei Wellenlängen (typisch 660 nm und 940 nm), die durch LEDs erzeugt werden, zeitlich schnell getaktet mit einem optischen Sensor aufzunehmen. Durch die schnelle Taktung kann das pulsierende Puls-Signal in einen variablen und einen Gleichanteil zerlegt werden. Der variable Anteil des Signals repräsentiert das pulsierende arterielle Blut. Durch die Analyse der Absorptionsverhältnisse der beiden Wellenlängen im variablen Signal, kann die Berechnung der Sauerstoffsättigung des arteriellen Blutes ($SpO_2$) erfolgen, da sich die Absorptionskoeffizienten für oxygeniertes und deoxygeniertes Hämoglobin unterscheiden und an den betrachteten Wellenlängen gegenläufig verhalten.

Durch die Transparenz des Gewebes in dem betrachteten Spektralbereich können bei starkem und vor allem variierendem Aussenlicht durch Änderungen Zusatzsignale erzeugt werden. Diese Einflüsse werden im Allgemeinen jeweils durch einen dritten Messpunkt ohne LED-Beleuchtung reduziert.

Diese Technologie ermöglicht es, kleine und sehr schnelle Sensoreinheiten zu konstruieren, die direkt an die üblichen Körperstellen (Muskeln) angebracht werden können.

Die Lichtquelle von Pulsoximetern ist bevorzugt ein integriertes multispektrales LED Modul, das als gepulste und steuerbare Beleuchtungseinheit dient. Die LEDs sind hierbei sehr schnell schaltbare variable Lichtquellen (typisch 10-5000 ps). Sie arbeiten ohne thermische Probleme mit hohen, aber für das Gewebe unkritischen Lichtleistungen. Erweiterte Pulsoximeter (z.B. die CO-Pulsoximeter der Fa. Masimo) verwenden zur zusätzlichen Analyse weiterer Hämoglobin-Derivate, wie Carboxyhämoglobin oder Methämoglobin, auch mehrere (bis zu 9) Wellenlängen im VIS- und NIR-Bereich. Solche erweiterten Pulsoximeter arbeiten ausschließlich mit Wellenlängen oberhalb von 600 nm.

Aus US 2012/0253153 A1 ist eine Vorrichtung mit den Merkmalen des Oberbegriffs von Patentanspruch 1 bekannt. Es handelt sich bei der bekannten Vorrichtung um ein Pulsoximeter, das ähnlich wie das zuvor beschriebene in seiner Funktionalität dahingehend erweitert ist, dass durch Hinzunahme weiterer Wellenlängen weitere Substanzen analysiert werden können. Die beschriebene Vorrichtung ist aber nicht zur Gewebe- oder Muskeloximetrie geeignet, die weiter unten beschrieben wird, da die Beleuchtungseinheit mit hohen Taktrate (Abtastrate) betrieben werden muss, die zur Erfassung der Pulsrate nötig ist, und bei dieser hohen Taktrate der Intensitätsverlauf als Funktion des Abstands zur Beleuchtungseinheit nicht annähernd mit hinreichender Auflösung erfasst werden kann. Der Intensitätsverlauf als Funktion des Abstands von der Beleuchtungseinheit wird zur Muskeloximetrie jedoch mit hoher Auflösung benötigt.

In der Spiroergometrie werden diverse Atemparameter bestimmt, um umfassende qualitative Aussagen über das muskuläre, zirkuläre und pulmonale System zu erhalten. Die zu bestimmenden Parameter sind hauptsächlich das pro Minute eingeatmete Sauerstoffvolumen, das Volumen des pro Minute ausgeatmeten Kohlendioxids und daraus abgeleitete Werte und Größen. Dabei sind die wichtigsten zu bestimmenden Größen für die Trainingssteuerung die $VO_2max$ und die ventilatorischen Schwellen (Ventilatory Threshold, VT1 und VT2), wobei $VO_2max$ die maximale Sauerstoffaufnahme des Sportlers ist.

Zur Bestimmung dieser Größen ist bei spiroergometrischen Messungen das Tragen einer Atemmaske durch den Probanden obligat. Zudem findet die Spiroergometrie meist unter definierten Umgebungsbedingungen in Laboren von Leistungszentren statt. Aufgrund der Komplexität der Geräte gestaltet sich eine Durchführung im Freien äußerst technisch und finanziell aufwendig. Daher kann die Datenerfassung nur bei geplanten leistungsdiagnostischen Untersuchungsterminen durchgeführt werden und ergibt so nur ein temporäres Bild vom Trainingszustand des Sportlers.

Bei spiroergometrischen Untersuchungen wird neben der Bestimmung der leistungsabhängigen Atemparameter des Probanden im Allgemeinen die Herzfrequenz (Pulsrate) mit bestimmt, die ebenfalls leistungsabhängig ist und mit einem weiteren Sensorsystem erfasst wird. Dieses kann zum Beispiel konventionell über ein EKG, einen Pulsmesser oder ein Pulsoximeter geschehen.

Zur Erfassung physiologischer Parameter außerhalb der medizinischen Anwendungen wurden in den vergangenen Jahren unterschiedliche neue Ansätze vorgestellt, um zunehmend unterschiedliche physiologische Parameter zu erfassen.

In US 2015/0282724 A1 wird zum Beispiel ein Kamerasystem vorgestellt, mit dem über die pulsatilen Veränderungen aus dem Bild-Signal die Pulsrate, die Herzfrequenzvariabilität (HRV) und die Atemfrequenz (RR) bestimmt wird. Dies lässt erkennen, dass unterschiedliche optische Sensorsysteme zur Bestimmung physiologischer Parameter eingesetzt werden können.

In US 2015/0282746 A1 wird ein System vorgestellt, bei dem mit einem üblichen Pulsoximeter mit zwei Wellenlängen neben der Pulsrate auch die Atemfrequenz über die Blutverschiebung im Körper durch den Atemzyklus erkannt und ausgewertet wird. Dass dieses gerade im Bereich des Sports mit hohen Signalintensitäten gut möglich ist, hat sich durch die Etablierung einiger klinischer Geräte mit dieser Technologie gezeigt. Eine vergleichbare original Messung und Auswertung ist auch in WO 2014/139830 A1 in Figur 7 gezeigt.

In US 2015/297133 A1 ist ein Verfahren dargestellt, das aus einer gemessenen Pulsrate (HR) und einer Atemfrequenz (RR) die ventilatorische Schwelle (VT) bestimmt. Dabei wird vorgeschlagen, die 2 Parameter aus zwei Sensoren, zum Beispiel einem EKG Sensor und einem Atmungssensor zu bestimmen. Hieraus ist zu ersehen, dass die Bestimmung einer ventilatorischen Schwelle (VT) nicht nur über spiroergometrische Methoden möglich ist, sondern auch über kontinuierliche mobile Messmethoden ohne Atemgasmessung realisiert werden kann.

Bei der Kombination der Informationen aus den zwei vorherigen Anmeldungen ist deutlich ersichtlich, dass der Ventilatory Threshold eines Sportlers mit einem einzigen optischen Sensor bestimmbar ist.

Die andere in der Leistungsdiagnostik konventionell eingesetzte Methode ist die Laktatschwellwertbestimmung. Diese wird im Allgemeinen punktuell und minimalinvasiv durchgeführt. Dabei wird die aerobe Schwelle häufig bei 2 mmol/l (Leistung bei 2 mmol/l Laktatkonzentration) angesetzt und die anaerobe Schwelle bei 4 mmol/l-Laktatkonzentration (Leistung bei 4 mmol/l Laktatkonzentration). Die anaerobe Laktatschwelle liegt bei vielen Sportlern je nach Leistungsfähigkeit im Bereich von 75-90% der maximalen Leistung. Die mittels Laktat zu bestimmenden Schwellen sind von weiteren äußeren Parametern abhängig (u.a. Wahl des Belastungsprotokolls, Trittfrequenz...) und bedingen tiefe Kenntnisse über die Verwendung diverser Schwellenwertmodelle.

Um diese Schwelle auch kontinuierlich und nichtinvasiv bestimmen zu können, wurde vorgeschlagen, diese über den pH Wert im Muskel zu bestimmen. Die optische Bestimmung des pH Wertes im Muskel wurde in "Noninvasive determination of exercise-induced hydrogen ion threshold through direct optical measurement", Babs R. Soller et al., J Appl Physiol 104: 837-844, 2008", in WO 2011/091280 A2 und in "Validation of a spectroscopic sensor for the continuous, noninvasive measurement of muscle oxygen saturation and pH", G. Ellerby et al., Physiol. Meas. 34 (2013) 859-871 vorgestellt. Dabei können die Parameter Muskeloxygenierung, Muskel pH Wert und Muskellaktatkonzentration aus denselben Messwerten bestimmt werden. Vorrichtungen und Verfahren zur Bestimmung der Muskeloxygenierung wurden hierzu in US 2008/097173 A1 und in WO 2010/053617 A2 offengelegt. Dies zeigt, dass die Muskeloxygenierung und die Muskellaktatkonzentration direkt ineinander überführt werden können und durch Messung der Oxygenierung die Laktatwertbestimmung zur Leistungsdiagnostik obsolet wird.

Eine Methode zur nichtinvasiven spektroskopischen Bestimmung des Laktatwertes im medizinischen Anwendungsbereich im Spektralbereich 1000 nm bis 1700 nm wurde in WO 2004/ 097365 A2 beschrieben. Darin sind auch viele wissenschaftliche Veröffentlichungen zu diesem Themenbereich der nichtinvasiven Laktatwertbestimmung zitiert.

Andere Vorrichtungen und Verfahren zu Bestimmung der Muskeloxygenierung während einer sportlichen Betätigung sind in WO 2010/093865 A1, WO 2013/158459 A1, US 2014/016132 A1 und US 2015/0196238 A1 dargestellt. Alle diese Systeme zeichnen sich durch multispektrale oder spektroskopische Vorrichtungen im NIR Spektralbereich aus, die weiterhin mit mindestens zwei Entfernungen zwischen der Beleuchtungseinheit(en)(Sender) und optischen Sensoren (Empfänger) die optischen Signale erfassen und daraus die physiologischen Werte bestimmen. Diese Technologien werden in der Fachwelt als NIRS (Near Infrared Spectroscopy) Methoden oder Gewebe- oder Muskel-Oximetrie bezeichnet. Bei US 2015/01962381 ist anzumerken, dass in die Sensoreinheit zur Aufnahme der physiologischen Signale drei Detektoreinheiten integriert werden sollen.

Im Unterschied zu den Pulsoximetern, die die pulsatilen Signalanteile aufnehmen und damit die arterielle Sauerstoffsättigung und die Pulsrate bestimmen, werden Gewebeoximeter verwendet, um die Sauerstoffsättigung des Hämoglobins in der Mikrozirkulation zu bestimmen. In der Mikrozirkulation erfolgt der Austausch des Sauerstoffs zwischen Blut und Gewebe-Zellen. Daher wird oft von der Gewebe-Sauerstoffsättigung gesprochen.

Die üblichen kommerziellen Gewebeoximeter (NIRS-Systeme) haben eine einfache NIR photometrische Aufnahme. Diese besteht, wie bereits beschrieben, typischerweise aus einer oder mehreren steuerbaren multispektralen LED Beleuchtungseinheiten und mehreren in unterschiedlichen Abständen angebrachten Fotosensoren. Dabei sind die Distanzen zwischen den Sendern und Empfängern im Allgemeinen im Bereich zwischen 1 cm und 10 cm angeordnet.

Der Einsatz von mehreren Detektoren (ortsaufgelöste Remissionsmessung) geht auf die Eigenschaften des Gewebes als streuendes und absorbierendes Medium zurück. Die ortsaufgelöste Messung hat den Vorteil, dass die Signatur der Licht-absorbierenden Bestandteile stärker wird, je größer der Abstand zwischen Emitter und Detektor ist. Dadurch wird mit dem größeren Abstand die Messung abgesichert, auch wenn durch die abfallende Intensität die Signalqualität schlechter ist als für den kurzen Abstand. Die ortsaufgelöste Messung erlaubt daher eine gewisse Trennung von Absorption und Streuung.

Bei diesen Vorrichtungen müssen die Stützwellenlängen sequentiell geschaltet werden und auch die unterschiedlichen Fotosensoren werden meist sequentiell ausgelesen. Durch die größeren Abstände zwischen Sender und Empfänger sind weiterhin die SignalIntensitäten häufig sehr niedrig, so dass mit verhältnismäßig langen Belichtungszeiten der Sensoren gearbeitet werden muss. Zusätzlich sind durch die relativ kleinen Unterschiede der Absorptionsquerschnitte des $HbO_2$ und HHb in dem typisch untersuchten Spektralbereich zwischen 700 nm und 950 nm ein sehr gutes Signal-Rausch-Verhältnis (SNR) der Messung nötig, welches üblicherweise eine größere zeitliche Glättung erfordert. So sind diese Sensoren nicht geeignet, die pulsatilen Signale zeitlich aufzulösen und können daher keine Informationen wie die Pulsfrequenz oder die arterielle Sauerstoffsättigung ($SpO_2$) zur Verfügung stellen.

Somit sind die Gewebeoximeter nicht zum Erfassen der in der Gesamtbeurteilung der physiologischen Signale des menschlichen Körpers eines Sportlers wichtigen Parameter geeignet. Die Systeme sind nicht pulsaufgelöst, das heißt sie können nicht Pulsrate, arterielle Sauerstoffzuführung, die Pulsintensität und die Atem-frequenz aufnehmen. So werden auch die ersten verfügbaren mobilen Geräte zur Bestimmung der Muskeloxygenierung häufig mit anderen Sensorsystemen zum Beispiel zum Erfassen der Pulsfrequenz parallel betrieben. Bei der Nutzung dieser Geräte ist die Kopplung unterschiedlicher Sensoren zur PR Messung aufwendig und so kann auch keine einheitliche Analysesoftware angeboten und eingesetzt werden. Als zusätzliche Nachteile sind zu sehen, dass die Sensoren die Signale von unterschiedlichen Stellen am Körper aufnehmen und auch eine genaue zeitliche Synchronisation der Messdaten durchgeführt werden muss.

Um ein differenzierteres Bild von einem Probanden punktgenau (zum Zeitpunkt des Trainings) zu erhalten, gilt es zu vermeiden, die beschriebenen konventionellen Methoden wie die ortsgebundene und aufwändige Spiroergometrie, die punktuelle Laktatdiagnostik oder die kontinuierliche und nichtinvasive Methode der NIRS mit unterschiedlichen Sensorsystemen an verschiedenen Stellen des Körpers, mit unterschiedlichen Konfigurationen, Auswertemethoden und Restriktionen einzusetzen.

Die bisher vorgestellten Systeme eigenen sich nicht zur kombinierten Messung der dargelegten Parameter in einem einzigen kompakten, mobilen Gehäuse für eine mobile Anwendung am Muskel des Sportlers. Dieses ist mit den bisher vorgestellten und dem Stand der Technik entsprechende Methoden nicht realisierbar, da zum einen eine hohe optische Signalqualität erreichbar werden muss, die Signale im gleichen Bereich des Gewebes generiert werden müssen und gerade zur Messung der Herzfrequenz hohe Taktraten erforderlich sind, um die Herzfrequenz sicher aus dem Photoplethysmogramm (auch Plethkurve, zeitlicher Verlauf des pulsierenden Hämoglobinsignals) zu bestimmen. Zusätzlich erschwert wird diese Anforderung durch Bewegungsartefakte der aktiven Muskulatur, die die Signale überlagern.

Ein einfaches Koppeln jeweils einer anderen Technologie oder der Kombination mehrerer bekannter Technologien erzeugt keine befriedigende Lösung.

Es ist daher eine Aufgabe der vorliegenden Erfindung, die Einschränkungen und Nachteile der vorhandenen Systeme zu vermeiden und insbesondere eine Vorrichtung und ein Verfahren zu schaffen, die unterschiedliche physiologische Parameter des Sportlers von Lunge, Herz und Muskeln unter Einbeziehung von Pulsoximetrie und Muskeloximetrie mit einer sehr kompakten Vorrichtung bestimmen, die - ohne den Trainingsbetrieb des Sportlers einzuschränken - kontinuierlich direkt durch Anbringung ausgerichtet auf die Körperoberfläche Daten erfasst.

Erfindungsgemäß werden diese und weitere Aufgaben durch eine Vorrichtung mit den Merkmalen des Patentanspruchs 1 und ein Verfahren mit den Merkmalen des Patentanspruchs 15 gelöst.

Erfindungsgemäß ist vorgesehen, dass als Fotosensor ein Kamerasensor auf CCD- oder CMOS-Basis mit einem 2-dimensionalen Array von Sensorelementen vorhanden ist. Der Kamerasensor ist so zur Beleuchtungseinheit angeordnet, dass er auf der Haut auf der gleichen Seite des Körperteils die Beleuchtungseinheit und benachbart zu dieser zur Auflage kommen kann, um durch Transflexion durch den Körper des Probanden von der Beleuchtungseinheit zum Kamerasensor gelangendes Licht erfassen zu können.

Die Datenverarbeitungseinheit ist dazu eingerichtet, den Kamerasensor in jeder Aktivierungssequenz zu den Aktivierungsstartzeitpunkten $t_k$ (k=1, 2 ... M) und über die jeweilige Aktivierungsdauer auszulesen, und die erfassten Intensitäten der Sensorelemente über Unterbereiche von Sensorelementen zusammengefasst und der jeweiligen Aktivierungssequenz aus der Folge n=1, 2 ... N von Aktivierungssequenzen und dem jeweiligen Aktivierungsstartzeitpunkt $t_k$ (k=1, 2 ... M) zugeordnet als Zeitreihen aufzuzeichnen. Die Zuordnung einer laufenden Nummer der Aktivierungssequenz und der jeweiligen Startzeitpunkte innerhalb jeder Aktivierungssequenz ist einer absoluten Zeitangabe zu jeder Aktivierung in jeder Aktivierungssequenz inhaltlich gleich, da sie ineinander umgerechnet werden können: dem Aktivierungsstartzeitpunkt $t_k$ in der n-ten Aktivierungssequenz ist folgende Zeit $\tau$ seit Messbeginn zugeordnet: $\tau(n, t_k) = n \cdot T_s + t_k$, wobei $T_s$ die Dauer einer Aktivierungssequenz ist und angenommen ist, dass die Aktivierungssequenzen ohne Unterbrechung aufeinanderfolgen.

Dabei ist die Datenverarbeitungseinrichtung dazu eingerichtet,die Intensitäten der Sensorelemente in Unterbereichen des Kamerasensors zusammenzufassen, indem Zeilen des Kamerasensors ausgelesen werden, die parallel zur Verbindungsachse zwischen Beleuchtungseinheit und Kamerasensor liegen. Da es eine Vielzahl von solchen Zeilen gibt, entlang derer die Entfernung von der Beleuchtungseinheit zu dem jeweiligen Sensorelement der Zeile des Kamerasensors zunimmt, stellt dies eine große Anzahl (gleich Zeilenanzahl) von zeitlich parallelen Messungen entlang der Zeilenrichtung dar. Solche zeitlich parallel ausgelesenen Zeilen können zu einer einzigen gemittelten Zeile zusammengefasst werden, wobei in der gemittelten Zeile die Intensitäten dann ein erheblich verbessertes Signal-Rausch-Verhältnis haben. Solche gemittelten Zahlen können für einzelne oder alle Wellenlängen gebildet werden. Durch die gemittelten Zeilen kann einerseits mit einer Taktfrequenz der Aktivierungssequenzen gearbeitet werden, die ausreichend hoch ist, um das Pulssignal des Kreislaufs aufzulösen. Andererseits kann durch die Mittelung über viele Zeilen des Kamerasensors ein ausreichend gutes Signal-Rausch-Verhältnis erreicht werden, sodass trotz der hohen Taktfrequenz die Intensitätsverläufe im NIR-Wellenlängenbereich im Verhältnis zueinander und als Funktion des Abstands von der Beleuchtungseinheit zur Muskeloximetrie ausgewertet werden können. Im Stand der Technik der Gewebeoximeter wurden zur Erzielung ausreichender Signal-Rausch-Verhältnisse hohe Belichtungszeiten eingesetzt, was die Auflösung des Pulssignals des Kreislaufs ausschloss.

Die Beleuchtungseinheit weist wenigstens einen LED-Typ mit Emissionsmaximum < 590 nm auf, vorzugsweise mit einem Emissionsmaximum bei 575 nm, wo sich die Absorptionskoeffizienten für oxygeniertes und deoxygeniertes Hämoglobin deutlich unterscheiden. Dies erlaubt eine zusätzliche Überprüfung, ob es sich bei einem vermeintlich als Pulssignal erkannten periodischen Signal tatsächlich um das Pulssignal des Kreislaufs handelt, da das echte Pulssignal mit einem entsprechenden Pulssignal für oxygeniertes Hämoglobin einhergeht. Damit kann das Pulssignal des Kreislaufs verlässlich von anderen periodischen oder näherungsweise periodischen Signalen (zum Beispiel Atmung oder periodische Bewegung beim Laufen) unterschieden werden.

In einer bevorzugten Ausführungsform ist vorgesehen, dass die Datenverarbeitungseinheit dazu eingerichtet ist, jeweils Zeilen des Kamerasensors, die parallel zur Verbindungslinie zwischen Beleuchtungseinheit und Kamerasensor verlaufen, auszulesen und alle Zeilen zu einem gemittelten Zeilenintensitätsverlauf

$$\bar{I}(i)_{\lambda m} = \frac{1}{N_Z} \sum_{a=0}^{N_Z} I_a(i)_{\lambda m}$$

zu mitteln, wobei i=1,2 ... $N_{sp}$ der durchlaufende Spaltenindex und $N_{sp}$ die Anzahl der Spalten des Kamerasensors ist und der Index Am die Wellenlänge des Emissionsmaximums des jeweiligen LED-Typs symbolisiert und als gemittelte Intensität $\bar{I}(i)_{\lambda m}(n, t_k)$ der jeweiligen Aktivierungssequenz n aus der Folge n=1, 2 ... N von Aktivierungssequenzen und dem jeweiligen Aktivierungsstartzeitpunkt $t_k$ (k=1, 2 ... M) zugeordnet als Zeitreihen aufzuzeichnen. Typische Kamerasensoren haben mehrere hundert Zeilen, sodass sich bei Mittelung über alle Zeilen ein sehr gutes Signal-Rausch-Verhältnis ergibt. Die Mittelung über alle Zeilen entspricht der Zusammenfassung der Intensitätswerte aller Sensorelemente jeweils jeder Spalte zu einem für jede Spalte gemittelten Intensitätswert.

Weiterhin ist es bevorzugt, dass die Datenverarbeitungseinheit dazu eingerichtet ist, die Intensitäten der Sensorelemente über alle Zeilen und Spalten zu einer über den Sensor integrierten Kamerasensorintensität

$$\bar{I}_{\lambda m} = \frac{1}{N_Z \cdot N_{Sp}} \sum_{a=0}^{N_Z} \sum_{i=0}^{N_{Sp}} I_a(i)_{\lambda m}$$

zusammenzufassen, wobei i=1,2 ... $N_{sp}$ der durchlaufende Spaltenindex, $N_{sp}$ die Anzahl der Spalten des Kamerasensors, a der durchlaufende Zeilenenindex, $N_z$ die Anzahl der Zeilen des Kamerasensors und der Index Am die Wellenlänge des Emissionsmaximums des jeweiligen LED-Typs symbolisiert, und diese der jeweiligen Aktivierungssequenz n aus der Folge n=1, 2 ... N von Aktivierungssequenzen und dem jeweiligen Aktivierungsstartzeitpunkt $t_k$ (k=1, 2 ... M) zuge-ordnet als Zeitreihen $\bar{I}_{\lambda m}(n,t_k)$ der Kamerasensorintensität aufzuzeichnen. Für jedes Emissionsmaximum steht so eine Zeitreihe $\bar{I}_{\lambda m}(n,t_k)$ zur Verfügung, die einzeln oder in Kombination ausgewertet werden kann.

In einer bevorzugten Ausführungsform ist vorgesehen, dass die Datenverarbeitungseinheit dazu eingerichtet ist, die Zeitreihen $\bar{I}_{\lambda m}(n,t_k)$ der Kamerasensorintensität der verschiedenen Wellenlängen λ1, A2 ... λL zu einer wellen-längenübergreifenden einzelnen Zeitreihe $\bar{I}(n,t_k)$ der Kamerasensorintensität zusammenzufassen und aufzuzeichnen.

Es ist weiter bevorzugt, dass die Beleuchtungseinheit wenigstens einen LED-Typ mit Emissionsmaximum im Bereich von 500 nm bis 540 nm und einen LED-Typ mit Emissionsmaximum im Bereich von 570 nm bis 585 nm umfasst. Insbesondere kann ein LED-Typ mit einer Wellenlänge von etwa 525 nm und ein LED-Typ mit einer Wellenlänge von 575 nm vorhanden sein. Diese Wellenlängen erlauben eine gute Diskriminierung zwischen oxygeniertem und deoxygeniertem Hämoglobin, da 525 nm ein isosbestischer Punkt ist, d.h. die Absorption von oxygeniertem im und deoxygeniertem im Hämoglobin ist gleich. Demgegenüber ist die Absorption von deoxygeniertem Hämoglobin bei 575 nm wesentlich kleiner als die von oxygeniertem Hämoglobin.

In einer bevorzugten Ausführungsform ist vorgesehen, dass die Beleuchtungseinheit wenigstens drei unterschiedliche LED-Typen umfasst, deren Emissionsmaxima einen NIR-Wellenlängenbereich von 650 nm bis 920 nm aufspannen. In diesen Wellenlängenbereich können Auswertungen zur Gewebeoximetrie und Muskeloxygenierung vorgenommen werden.

Vorzugsweise weist die Beleuchtungseinheit in dem aufgespannten NIR-Wellenlängenbereich wenigstens vier unterschiedliche LED-Typen mit Emissionsmaxima verteilt in dem aufgespannten NIR-Wellenlängenbereich auf.

In einer bevorzugten Ausführungsform ist vorgesehen, dass die Beleuchtungseinheit im NIR-Wellenlängenbereich eine Mehrzahl von LED-Typen mit Emissionsmaxima verteilt in dem Wellenlängenbereich 800-1100 nm aufweist, einschließlich eines LED-Typs mit Emissionsmaximum bei 960 nm, eines LED-Typs mit Emissionsmaximum bei 930 nm und wenigstens zweier LED-Typen mit Emissionsmaxima im Bereich von 790-920 nm, und dass die Datenverarbeitungseinheit dazu eingerichtet ist, aus der bei 960 nm gemessenen Absorption die Konzentration von Wasser unter Berücksichtigung der Absorption durch Fett bei 930 nm und der Absorption durch Hämoglobin durch Auswertung wenigstens zweier Absorptionen an Stützstellen im Bereich 790-920 nm zu bestimmen, vorzugsweise mit wenigstens zwei Stützstellen im Bereich 800-910 nm.

In einer bevorzugten Ausführungsform ist vorgesehen, dass die Datenverarbeitungseinheit dazu eingerichtet ist, die Zeitreihe der wellenlängenübergreifenden Kamerasensorintensität $\bar{I}(n,t_k)$ einer Fourier-Transformation zu unterziehen und zur Erkennung des Pulssignals eine Verteilungsspitze bei einer Grundfrequenz zu suchen, die von einer oder mehreren Verteilungsspitzen von Oberwellen bei ganzzahligen Vielfachen der Grundfrequenz begleitet ist.

In diesem Zusammenhang ist es bevorzugt, dass die Datenverarbeitungseinheit dazu eingerichtet ist, nach Erkennung eines Pulssignals die Richtigkeit der Erkennung zu bestätigen, wenn in dem Signal der Kamerasensorinten-sität $\bar{I}_{\lambda m}(n,t_k)$ mit Am aus dem Wellenlängenbereich von 570-585 nm ein stärkeres pulsierende Signal mit der Grundfrequenz als in dem Signal der Kamerasensorintensität $\bar{I}_{\lambda m}(n,t_k)$ mit Am aus dem Wellenlängenbereich von 500-540 nm auffindbar ist. Wie oben erläutert ist die Absorption von oxygeniertem Hämoglobin im Wellenlängenbereich 570-585 nm wesentlich größer als der von deoxygeniertem Hämoglobin, sodass sich das Puls-signal im Wellenlängenbereich 570-585 nm klarer ausprägt.

Dabei ist es weiterhin bevorzugt, dass die Datenverarbeitungseinheit dazu eingerichtet ist, in dem Fourier-Spektrum der wellenlängenübergreifenden Kamerasensorintensität ein dynamisches Bandpass-Filter für die Pulssignalerkennung anzuwenden, wobei das Zentrum des akzeptierten Frequenzbandes bei der erkannten Grundfrequenz liegt und die Breite des Frequenzbandes vorgegeben ist, wobei das dynamische Bandpass-Filter Änderungen der erkannten Grundfrequenz des Pulssignals folgt, wenn die geänderte Grundfrequenz innerhalb des akzeptierten Frequenzbandes liegt, und wobei ein hypothetisch neu erkanntes Pulssignal mit seiner Grundfrequenz verworfen wird, wenn es außerhalb des akzeptierten Frequenzbandes liegt. Dieser Test basiert auf dem Umstand, dass sich die Pulsfrequenz nicht sprunghaft ändert, sodass bei gleitender Änderung der Pulsfrequenz an eine sich tatsächlich ändernde Puls-frequenz des Probanden an das dynamische Bandpass-Filter der Änderung folgt und nur vermeintlich erkannte Pulssignal verworfen, bei denen eine zu große sprunghafte Änderung des vermeintlich neuen Pulssignals gegenüber dem zuletzt erkannten auftritt.

In einer bevorzugten Ausführungsform ist vorgesehen, dass die Datenverarbeitungseinheit dazu eingerichtet ist, im Fourier-Spektrum der wellenlängenübergreifenden Kamerasensorintensität eine Verteilungsspitze mit einer Grundfrequenz, die keine begleitenden Oberwellen mit Verteilungsspitzen bei ganzzahligen Vielfachen der Grundfrequenz hat und die kleiner als die Frequenz des Pulssignals ist, der Atmung zuzuordnen und daraus die Atemfrequenz zu bestimmen.

In diesem Zusammenhang ist bevorzugt, dass die Datenverarbeitungseinheit dazu eingerichtet ist, im Fourier-Spektrum zur Atemsignalerkennung ein dynamisches Bandpass-Filter anzuwenden, wobei das Zentrum des Frequenzbandes bei der erkannten Atemfrequenz liegt unter die Breite des Frequenzbandes vorgegeben ist, wobei das dynamische Bandpass-Filter Änderungen der erkannten Atemfrequenz folgt, wenn die geänderte Atemfrequenz innerhalb des akzeptierten Frequenzbandes liegt und wobei ein hypothetisch neu erkanntes Atemsignal mit seiner neuen Atemfrequenz verworfen wird, wenn diese außerhalb des akzeptierten Frequenzbandes liegt.

In einer bevorzugten Ausführungsform ist vorgesehen, dass die Datenverarbeitungseinheit dazu eingerichtet ist, die Zeitreihen $\bar{I}(i)_{\lambda m}(n,\ t_k)$ der Kamerasensorintensität für Emissionsmaxima Am im NIR-Wellenlängenbereich im Verhältnis zueinander und in ihren Abhängigkeiten von der Entfernung von der Beleuchtungseinheit (gegeben durch den Spaltenindex $i = 1,\ 2 \dots N_{sp}$, d.h. die Entfernung nimmt mit zunehmendem Spaltenindex $i$ zu) auszuwerten, um daraus den Oxygenierungsgrad des Muskels zu bestimmen.

Zur Erläuterung der Grundlagen der vorliegenden Erfindung muss an dieser Stelle etwas weiter und tiefer in wissenschaftlichen Grundlagen der Gewebespektroskopie, der physiologischen Messtechnik und in die spezifischen Eigenschaften heute zugänglicher optoelektronischer Systemkomponenten eingegangen werden. Es ist bekannt, dass das Licht auf dem Weg durch das Gewebe zum einen durch Streuung und zum anderen durch Absorption abgeschwächt wird. Da die Streuung im Gewebe im konventionellen Spektralbereich der NIRS relativ konstant ist, ist es sinnvoll solche zusätzliche Spektralbereiche in Betracht zu ziehen, bei denen die Absorptionen gerade des Hämoglobins wesentlich höher sind. Wenn der Anteil der Absorption relativ zur Streuung höher ist, so sind die pulsatilen Anteile - gerade die des Hämoglobins - zur Erfassung der Hämoglobinänderungen durch den Herzschlag oder die Atmung stärker. Dieses ist im visuellen Spektralbereich (VIS), gerade zwischen 500 nm und 600 nm gegeben. Ein weiterer Vorteil durch die stärkeren Absorptionsquerschnitte ist, dass die Strahlung nicht so tief in das Gewebe eindringt, und somit die optischen Signale nur aus dem direkten fein strukturierten Gewebe stammen und im Allgemeinen keine größeren Gefäße im Messvolumen erfasst werden. Da die größeren Gefäße bei der Muskelkontraktion mehr verändert werden als das durch Mikrozirkulation gekennzeichnete direkte Muskelgewebe, sind durch diesen Effekt schwächere Bewegungsartefakte zu erwarten. Ein weiterer Vorteil beim Einsatz dieses Spektralbereichs ist die hier signifikante spektrale Charakteristik des oxygenierten und des deoxygeniertem Hämoglobins ($HbO_2$ und $HHb$). Somit kann in diesem Bereich mit hohen Signalintensitäten spezifisch die Pulsation des oxygenierten und deoxygeniertem Hämoglobins untersucht werden.

Die theoretische Grundlage für die spektroskopischen oder photometrischen Untersuchungen ist das Beer-Lambertsche Gesetz. Mit diesem können Konzentrationen $c_i$ von absorbierenden Molekülen in Lösungen bei dem Durchtritt von Licht bestimmt werden.

$$I_\lambda = I_{0,\lambda} e^{-\mu_{a,\lambda} \cdot l_\lambda} \qquad (1)$$

Mit $I_\lambda$ der Lichtintensität nach Durchtritt durch die zu untersuchenden Substanz, $I_{0,\lambda}$ der eingestrahlten Lichtintensität, $\mu_{a,\lambda}$ dem wellenlängenabhängigen ($\lambda$) Gesamtabsorptionskoeffizienten und der Weglänge $l$ durch die Substanz. Durch die streuenden Eigenschaften von Gewebe ist hier mit einer effektiven Weglänge zu rechnen, die im Allgemeinen auch wellenlängenabhängig ist, was in diesem Spektralbereich (500-1000 nm) und Anwendungsfall allerdings vernachlässigt werden kann. Alternativ kann ein zusätzlicher wellenlängenabhängiger Streukoeffizient genutzt werden.

Durch Umformung erhält man die Absorption

$$A_\lambda = ln\left(\frac{I_\lambda}{I_{0,\lambda}}\right) = -l_\lambda \cdot \mu_{a,\lambda} \qquad (2)$$

Dieses allgemeine Gesetz muss nun weiter diversifiziert werden, da eine Substanz wie zum Beispiel menschliches Blut oder das menschliche Muskelgewebe aus vielen chemischen Teilsubstanzen (molekularen Verbindungen) besteht und deren Absorptionskoeffizienten sich wellenlängenabhängig unterscheiden. Bei den Substanzen erhält man

$$\mu_{a,\lambda} = \sum_{i=1}^{n} \varepsilon_{i,\lambda} \cdot c_i \qquad (3)$$

Für m Wellenlängen kann dies nun mit der Annahme, dass die Weglängen für alle Wellenlängen gleich bleiben, wie folgt umgeformt werden:

$$
\begin{bmatrix} \ln\left(\frac{I_{\lambda 1}}{I_{0,\lambda 1}}\right) \\ \vdots \\ \ln\left(\frac{I_{\lambda m}}{I_{0,\lambda m}}\right) \end{bmatrix} = -l \begin{bmatrix} \varepsilon_{1,\lambda 1} & \cdots & \varepsilon_{n,\lambda 1} \\ \vdots & \ddots & \vdots \\ \varepsilon_{1,\lambda m} & \cdots & \varepsilon_{n,\lambda m} \end{bmatrix} \begin{bmatrix} c_1 \\ \vdots \\ c_n \end{bmatrix} \qquad (4)
$$

Die einzelnen Extinktionen, die durch die Matrix $[\varepsilon_{n,\lambda m}]$ beschrieben sind, ergeben zusammen die Gesamt-Absorptionsmatrix $\boldsymbol{E}_\lambda$. Damit kann diese Beziehung in folgender Form geschrieben werden:

$$
\boldsymbol{A}_\lambda = -l\boldsymbol{E}_\lambda\boldsymbol{C} \qquad (5)
$$

oder

$$
\boldsymbol{C} = -\frac{1}{l}\boldsymbol{E}_\lambda^{-1}\boldsymbol{A}_\lambda \qquad (6)
$$

Damit lassen sich die Konzentrationen der Substanzen mit bekannten Extinktionskoeffizienten $\boldsymbol{E}_\lambda$, der gemessenen Intensität $\boldsymbol{I}_\lambda$ und der bekannten eingestrahlten Lichtintensität $\boldsymbol{I}_{0,\lambda}$ bestimmen.

Für die physiologische Diagnostik eines Sportlers sind hier weitestgehend das oxygenierte und deoxygenierte Hämoglobin wichtig, allerdings müssen auch die Einflüsse von Melanin (Hautfarbstoff, Wasser und Fett (subkutane Fettschicht) berücksichtigt werden.

Bei der Messung der Hämoglobinkonzentrationen im Gewebe muss weiterhin festgehalten werden, dass sich der Puls des Herzschlages durch die Arterien mit einem hohen Anteil des oxygenierten Hämoglobins (HbO$_2$, typisch 98%) ausbreitet. Somit ist gerade der Puls nicht nur durch eine generelle Veränderung des Hämoglobingehaltes im Muskel gekennzeichnet sondern zusätzlich speziell durch die (systolisch bedingte) Erhöhung des oxygenierten Hämoglobins. Im Gegensatz dazu unterscheiden sich relative Konzentrationsänderungen des Hämoglobins, die durch Bewegungen, Atmungs und Muskelanspannungen verursacht werden insofern, als dass sich hier die Konzentrationen gleichverteilt verschieben.

So kann bei dem Einsatz von Wellenlängen, bei denen das Hämoglobin isosbestische Absorptionskoeffizienten (z.B. ca. 520 nm) aufweist und von Wellenlängen, bei denen das HbO$_2$ deutlich höhere Extinktionskoeffizienten als das HHb besitzt, mathematisch eine genaue Differenzierung durchgeführt werden. Als typische Wellenlänge ist hier 575 nm zu nennen.

Ein weiterer Vorteil ist, dass das Pulssignal stabiler erfasst werden kann, da die durchschnittliche Differenz des Signals zwischen Pulsmaximum und Pulsminimum im sichtbaren Spektralbereich im Verhältnis zum Grundsignal im Bereich 575 nm deutlich höher ist als im VNIR-Spektralbereich. Dieser Unterschied kann bis zu Faktor 5 größer sein.

Der Einsatz dieser Wellenlängen zur pulsaufgelösten Analyse widerspricht aber grundlegend den Anforderungen der konventionellen NIRS Systeme. So müssen konventionelle NIRS Systeme mit niedrigeren Absorptionskoeffizienten üblicherweise mit größeren Weglängen im Gewebe arbeiten. Weiterhin müssen in der NIRS Technologie im Allgemeinen mehrere spektrale Stützpunkte, dafür aber mit niedrigerer Frequenz, jedoch höherer Präzision erfasst werden.

Da die zusätzliche Pulsüberwachung bislang nur über den Einsatz von unterschiedlichen Sensorsystemen an unterschiedlichen Messstellen im Gewebe zu erreichen ist, wird hier ein neuer Lösungsansatz vorgeschlagen.

In der Kameratechnologie haben sich hochintegrierte miniaturisierte digitale CMOS Kamerasensoren etabliert. Diese Sensoren werden zumeist mit einer abbildenden Optik zur Erzeugung von zweidimensionalen Bildern eingesetzt. Diese CMOS Bildsensoren mit der Möglichkeit der kontinuierlichen schnellen Bilderzeugung für digitale Videosysteme sind Stand der Technik und finden vielseitige Anwendung z.B. in mobilen Multifunktionsgeräten (Smartphones), der Automatisierungstechnik, Qualitätskontrolle, Medizintechnik und Mikroskopie.

Diese Kamerasensoren haben 0,5-5 Millionen strukturierte Lichtempfänger. Weiterhin sind in diesen Sensoren miniaturisiert auch einstellbare Verstärkerschaltungen, Digitalisierungselemente (A/D-Wandler), Dunkelpixel und andere vorteilhafte Funktionen integriert. Weiterhin kann mit CMOS Sensoren der neueren Generationen ein interner Schwarzwertabgleich erfolgen. An den Rändern sind Pixel schwarz abgedeckt. Diese werden zusätzlich ausgelesen und intern für eine Schwarzwertnormierung eingesetzt. Dies behebt zwar nicht das Problem von Fremdlichteinflüssen, aber die üblichen Probleme, wie der Drift der Sensorsignale bei Temperaturschwankungen oder Schwankungen in der Versorgungselektronik. Damit können Bilder mit sehr kurzen Belichtungszeiten und hohen Lichtintensitäten aufgenommen werden. Folglich sind Fremdlichteinflüsse generell niedrig. Falls Fremdlichteinflüsse auftreten, kann jeweils zusätzlich ein Hintergrundbild ohne LED-Beleuchtung und mit stark reduzierter ROI (region of interest; untersuchter Bildbereich) aufgenommen werden, um die Bilderfassung zusätzlich zu optimieren.

Die CMOS Sensoren sind mit typischen Bildkantenseiten von 3-10 mm sehr kompakt gebaut und benötigen wenig Raum für zusätzliche Elektronik, die bei dem Einsatz konventioneller Photodetektoren nötig wäre. Durch eine weiterhin integrierte Logik lassen sich diese Bauteile umfangreich parametrisieren. Bei sehr spezieller Parametrisierung lassen sich solche Pixelarrays oder CMOS Kamerasensoren sehr vorteilhaft für ein hier beschriebenes Sensorsystem einsetzen. Ein weiterer Vorteil bei der Verwendung von Sensorarrays und insbesondere von CMOS-Sensorarrays ist deren hohe Verfügbarkeit. Zweidimensionale Sensorarrays erlauben insbesondere auch eine höhere Messgeschwindigkeit bei gleichzeitig besseren Signal-Rausch-Verhältnissen. Die digitalen Daten können sehr schnell sequentiell oder parallel an einen Prozessor übertragen werden, wobei viele kommerzielle Prozessoren schon direkt für den Betrieb mit digitalen CMOS Kameraarrays ausgerüstet sind.

Die sehr gute Bildqualität und den niedrigen Lichtbedarf dieser Sensoren (hohe Quanteneffizienz bei typisch über 50%) erlauben den Einsatz von kleinen, miniaturisierten Beleuchtungseinheiten.

Ein solches Sensorarray ermöglicht es typischerweise 0,5-5 Millionen Bildpunkte (Pixel) mit einer Belichtung einer LED aufzunehmen. Jeder einzelne Bildpunkt ist dabei ein Pixel, welches eine Intensitätsinformation in üblicherweise 10, 12, 14 oder 16 Bit Datentiefe enthält. Da diese sehr hohe Pixelanzahl für diese Anwendung nicht benötigt wird, kann die Datenmenge durch unterschiedliche Funktionen des Sensors und durch die Auswahl eines Teilbereichs auf zum Beispiel ca. 500 x 50 Bildpunkte stark reduziert werden. Dieses wird ermöglicht, indem die bei den Kamerasensoren mögliche Teilbildaufnahme eingesetzt wird, um die Aufnahme zu beschleunigen. Bei CMOS-Sensoren können Teilbilder (regions of interest - ROI) eingestellt werden, die es ermöglichen, nur den eingestellten, interessierenden Bildbereich des Sensors bei gleichzeitiger Beibehaltung der Grunddatenrate auszulesen. Weiterhin kann durch die Methode des Binnings (Zusammenfassen von Pixeln) oder Skippings (Überspringen von Pixeln) die Auflösung weiter reduziert werden.

Diese digitalen optischen Daten können dann zu einem Prozessor übertragen werden und dort weiter auf ca. 50 entfernungsabhängige Intensitätswerte mit einem sehr guten Signal-Rausch-Verhältnis aufintegriert werden.

Durch die reduzierte Datenmenge kann der Sensor in einer höheren Bildrate betrieben werden. So können hier typisch bis zu 200 bis 500 Bilder pro Sekunde generiert werden. Die hohe Bild-Frequenz kann genutzt werden um das durch den Puls zeitlich veränderliche optische Signal mit hoher Abtastrate aufzunehmen. Dabei ist auf die Einhaltung des Nyquist-Shannon-Abtasttheorems zu achten, wobei ein Zeitsignal mit mindestens dem Doppelten der höchsten vorkommenden Frequenz abgetastet werden muss. Da ein Maximal-Puls eines Sportlers mit 200 Schlägen/min, also ca. 3 pro Sekunde anzunehmen ist, ist mit dem erfindungsgemäßen Aufnahmeprinzip das Abtasttheorem erfüllt und darüber hinaus eine sehr gute pulsaufgelöste Erfassung mit gleichzeitig sehr hoher Datentiefe möglich.

Ein typischer Kamerasensor kann beispielsweise 1280 x 960 Sensorelemente, d.h. 960 übereinander liegende, parallele Zeilen mit einer Länge von jeweils 1280 Sensorelementen. Die Zeilen verlaufen parallel zur Verbindungslinie zwischen Beleuchtungseinheit und Kamerasensor. Bei voller Auflösung, d.h. Erfassung aller Sensorelemente, ist die Bildrate auf 45 fps (frames per second) begrenzt. Mit einer eingeschränkten Bildgröße von 1280 x 100 Sensorelementen (d.h. es werden nur 100 übereinander liegende Zeilen ausgelesen, die einen mittleren Streifen auf der Kamera Sensorfläche bilden, während in Zeilenrichtung die volle Auflösung erhalten bleibt) kann eine Bildrate von 346 fps erreicht werden. Das Auslesen von mindestens 100 Zeilen ist bevorzugt.

Die gute zeitliche Auflösung wird benötigt, um aus der aufgenommenen Intensitätskurve die Puls-basierenden Parameter wie die Herzfrequenz HR, die Pulsstärke PI oder die Atemfrequenz RR zu bestimmen. Gerade Bewegungsartefakte sind durch kurzzeitige (höherfrequente) Signalanteile gekennzeichnet. Daher ist eine ausreichend hohe Abtastrate hilfreich um auch diese Artefakte vom Primärsignal (Pulskurve) trennen zu können.

Bei der Betrachtung der Kamerasensoren müssen auch die spektralen Empfindlichkeiten berücksichtigt werden. Sehr wichtig sind hier gerade der visuelle (VIS, ca. 400 nm bis 650 nm) und nahe Infrarotbereich (NIR, ca. 650 nm bis 1200 nm). Der NIR Spektralbereich ist das optische Fenster des menschlichen Körpers, also der Bereich in dem optische Strahlung tiefer in das Gewebe eindringen kann. Die Messung im VIS-Bereich wiederum ermöglicht keine großen Eindringtiefen, aber zeichnet sich durch starke Pulsation aus, da hier das Hämoglobin sehr stark absorbiert. Viele verfügbare Kamerasensoren (CMOS oder CCD) sind gerade in diesen Spektralbereichen empfindlich.

Erfindungsgemäß werden die zweidimensionalen Sensorarrays nicht verwendet, um eine ortsaufgelöste Bildaufnahme in zwei Dimensionen durchzuführen, was die häufigste Anwendung von 2D-Arrays aus Sensorelementen ist. Vielmehr werden die Sensorelemente in einer Sensorrichtung zur Erfassung der entfernungsabhängigen Signale verwendet um die Lichtintensität in Abhängigkeit von der Weglänge durchs Gewebe zu bestimmen (ortsaufgelöste Remissionsmessung). Die andere Dimension dient zur Integration der Signale der einzelnen Photozellen zur Verbesserung des Signal-Rausch-Verhältnisses. Somit kann eine sequentielle Integration der optischen Signale mit aufeinander folgenden Belichtungszeiten in eine parallele Integration mit nur einem Lichtpuls pro definierter Wellenlänge und auch nur einem Lichtdurchtritt durchs Gewebe realisiert werden.

Das Verfahren ermöglicht die Verwendung nebeneinander liegender Spalten, um pro Zeiteinheit mehr Photosignale mit einer Belichtung zu erzeugen und damit optimierte Signale zu erhalten. Dieses wird insbesondere durch die sehr homogenen Streueigenschaften des Gewebes ermöglicht. Unter paralleler Messung wird hier eine quasi gleichzeitige Messung verstanden. Selbstverständlich ist klar, dass die einzelnen Pixel und Zeilen des Sensors sequenziell ausgelesen werden. Die Abtastfrequenz ist aber so hoch (typisch >40MHz Pixeltakt), dass von einer quasi synchronen Messung der parallelen Zeilen gesprochen werden kann, insbesondere da die Belichtung der Photoempfänger quasi zeitgleich auftritt. Damit wird das Auslesen von Teilbildern mit höheren Geschwindigkeiten erreicht. Bessere Signal-Rausch-Verhältnisse sind damit in der optischen Datenaufnahme erzeugbar.

Dank dieser Anordnung können das Beleuchtungssystem (z. B. eine miniaturisierte, multispektrale LED Beleuchtungseinheit) - und das optische Empfängersystem direkt im Sensor an einer Messstelle integriert werden. Die Beleuchtung und der Kamerasensor können direkt am Messbereich am Muskel angebracht werden. Damit kann auf relativ starre und große optische Einrichtungen zur Übertragung des Lichtes verzichtet werden. Weiterhin ist die Beleuchtung auf diese Weise wesentlich höher. Dieses wiederum erhöht die Lichtausbeute, so dass die Belichtungszeiten sehr kurz gehalten werden können und der Anteil von Messlicht trotz möglichem Fremdlicht sehr hoch ist.

Die ganze Anordnung enthält somit nur eine multispektrale LEDbasierte Beleuchtungseinheit und das hochintegrierte CMOS Sensor-array. Somit kann das optische Modul sehr klein (bevorzugt kleiner 10 mm x 20 mm x 5 mm, typischerweise etwa 10 mm x 10 mm x 1,5 mm) ausgebildet werden.

Neben dem optischen Modul muss der Sensor ein weiteres Prozessormodul enthalten. Dieses Prozessormodul kann entweder mit dem optischen Modul integriert werden oder kann über ein dünnes Kabel separat vom optischen Modul ausgelegt werden. Es ist von großem Vorteil, wenn die optische Sensorik sehr klein und direkt am Muskel platziert werden kann. Damit kann der Sportler während des Trainings ohne Einschränkungen des Trainingsprozesses die physiologischen Daten erfassen.

Es ist auch möglich, das optische Sensormodul direkt in eine Textilie des Sportlers einzubringen, so dass der Sensor nicht separat am Muskel befestigt werden muss.

Eine solche Vorrichtung kann daher auch mit einer funkbasierten Datenübertragung (zum Beispiel Bluetooth, WIFI oder ANT+) und einer integrierten Energieversorgung (Akku oder Batterie) ausgestattet werden und direkt am Muskel des Sportlers befestigt werden. Auf Glasfasern oder andere optische Übertragungselemente (Linsen etc.) kann verzichtet werden. Aufgrund der Bauformen der CMOS Anordnungen von wenigen Millimetern haben diese ausreichend Platz in einem Miniatursystem.

Bevorzugt weist die Vorrichtung ein Gehäuse auf und ist als kompakte Baueinheit so ausgebildet, dass sie gut an einem Muskel des Sportlers platziert werden kann und so auch während der sportlichen Betätigung keinen störenden Einfluss auf den Trainingsprozess hat. Die kompakte Baueinheit enthält wenigstens die multispektrale Lichtquelle und das Sensor-Array, einen Prozessor zur Datenauswertung und Speicherung, eine elektrische Versorgungseinheit und eine Vorrichtung zur kabelgebundenen oder kabellosen Datenübertragung.

Es ist aber auch möglich, die optische Einheit bestehend aus multispektraler LED und CMOS Sensor Array separat in ein Gehäuse zu integrieren. Die rechnerische Auswertung kann dann in einer von der Vorrichtung getrennten Auswerteeinrichtung erfolgen. Wenn in der erfindungsgemäßen Vorrichtung die Messwerte digitalisiert und über eine elektrische Verbindung an eine zentrale Auswerteinheit übergeben werden, können die Übertragungskabel dünn gehalten werden. Gleichzeitig ist es nicht erforderlich, den Sensor bzw. die erfindungsgemäße Vorrichtung aufgrund von notwendigen Rechnern, Ein- oder Ausgabegeräten so gross auszubilden, dass ein Anbringen an den Messorten nicht mehr möglich ist. Eine externe Auswerteeinrichtung bietet auch die Möglichkeit der temporären Datenspeicherung oder der Datenauswertung mittels komplexeren, mathematischen Verfahren.

Mit der vorliegenden Erfindung kann anhand rein optischer Messungen und Auswertungen ein umfassendes physiologisches Bild der Reaktion des Sportlers auf eine muskuläre Beanspruchung generiert werden. Zum Einsatz in der Leistungsdiagnostik (z. B. Sauerstoffsättigung im Muskelgewebe) wird bevorzugt Licht im sichtbaren (VIS) und nahinfraroten (NIR) Spektralbereich, insbesondere im sehr nahen Infrarotbereich, z. B. im VNIR-Bereich von 500 nm bis 1000 nm, eingesetzt. Dieses Licht wird vorzugsweise durch das beschriebene integrierte multispektrale LED Beleuchtungsmodul erzeugt.

Physiologische Blut- und Gewebewerte sind im Rahmen der vorliegenden Beschreibung alle Werte, welche sich bei einem Sportler zu leistungsdiagnostischen Zwecken oder zur Überwachung des Trainingsablaufs ermitteln lassen. Dies gilt insbesondere für die nachstehend bereits etablierten Parameter.

Die Parameter, die mit der Vorrichtung und dem Verfahren direkt bestimmt werden können, sind zum Beispiel:

Pulsfrequenz (HR)

Pulsform und Struktur

Herzfrequenzvariabilität (HRV)

PI (Puls oder Perfusionsindex)

Atemfrequenz (RR)

Muskelsauerstoffsättigung (SmO$_2$)

Gewebehämoglobinindex (THI)

Konzentration des desoxygenierten Hb im Gewebe (cHHb)

Konzentration des oxygenierten Hb im Gewebe (cHbO$_2$)

Konzentration H$_2$O (cH$_2$O)

Arterielle Sauerstoffsättigung (SpO$_2$)

Für die weiteren Betrachtungen ist es hilfreich, bei den Parametern die zeitlichen Puls-basierten und die spektralen Absorptions-basierten voneinander abzugrenzen. Die Puls-basierten (pulsatilen) Parameter werden aus der zeitlich veränderlichen Pulskurve (Plethysmogramm) errechnet. Dazu gehören die Parameter HR, HRV, PI, RR und die Pulsform- und Struktur. Die Absorbtions-basierten Messwerte errechnen sich aus den die verschiedenen Intensitäts- bzw. Absorbtionsverhältnissen an den spektralen Stützstellen. Dazu gehören die Parameter SmO$_2$, THI, cHHb, cHbO$_2$ und cH$_2$O.

Durch die zeitabhängige Auswertung kann zwischen Informationen vom Gewebe und arteriellem Blut unterschieden werden.

Zur Bestimmung der arteriellen Sauerstoffzuführung kann die Berechnungen der prozentualen SpO$_2$-Konzentration wie in "The light-tissue interaction of pulse oximetry" (Mannheimer Ph.D.; Anesth. Analg. 2007 Dec; 105(6 Suppl): S10-7. Review) durchgeführt werden. Bei der Auswertung können vergleichbar zu der konventionellen Oximetrie zwei Spektralbereiche verglichen werden. Dafür können die konventionellen Wellenlängen von 660 nm und 910 nm eingesetzt werden, es kann aber auch der pulsatile multispektrale Ansatz genutzt und zusätzliche NIR-Wellenlängen zur Auswertung eingesetzt werden.

Die Berechnung der Parameter aus den optischen Signalen kann direkt in dem integrierten Prozessor des Sensorsystems durchgeführt werden.

Wie in der Beschreibung dargelegt, können aus diesen Messwerten mit unterschiedlichen mathematischen Verfahren einige der Parameter berechnet und abgeleitet werden, die heute durch die Leistungsdiagnostik bestimmt werden. Somit können mit der vorliegenden Erfindung die heutigen üblichen leistungsdiagnostischen Tests durch ein einzelnes Sensorsystem abgelöst oder ergänzt werden, welches im Gegensatz zu den aktuellen Methoden nichtinvasiv ist, kontinuierlich misst und auch während des normalen Trainings ohne wesentliche Einschränkungen vom Sportler benutzt werden kann.

Der Bildsensor kann bevorzugt zusammen mit der Auswerteeinrichtung und gegebenenfalls einer Steuereinrichtung sowie gegebenenfalls einer Speichereinrichtung für Referenzdaten bereits in einem Halbleiterbauelement monolithisch integriert werden, so dass ein kompakter und kostengünstiger Aufbau möglich ist und aufwendige zusätzliche Verdrahtungen entfallen oder noch geringer gehalten werden können.

Es ist wichtig festzuhalten, dass nicht nur eine einzelne Messvorrichtung an einer festgelegten Körperstelle positioniert werden kann, sondern dass es durch den Einsatz mehrerer Messvorrichtungen auch möglich ist, parallel an unterschiedlichen Messstellen Daten zu erfassen. So können Missverhältnisse zwischen z.B. der rechten und der linken Körperseite untersucht werden (z.B. im Radsport bei der Bestimmung des Ermüdungsgrades beider Beine). Ein häufiger Einsatzzweck ist die Platzierung eines Sensors an der beanspruchten Muskulatur und eines weiteren Sensors zentral an der Brust oder der Schulter, um Oxygenierungsdifferenzen und zeitliche Verteilungsmuster zu untersuchen. Eine weitere häufig geforderte Kombination ist der Vergleich der Messwerte zwischen Muskel und Gehirn durch eine Muskel-Stirnschaltung.

Die Erfindung wird im Folgenden anhand von Ausführungsbeispielen in den Zeichnungen näher erläutert, in denen

Fig. 1 eine schematische perspektivische Darstellung der erfindungsgemäßen Vorrichtung von einem Standpunkt innerhalb des beobachteten Körperbereichs ist,

Fig. 2 ein Blockschaltbild zur Erläuterung des Aufbaus und der Funktionsweise der Vorrichtung zeigt,

Fig. 3 eine schematische Schnittansicht durch eine erfindungsgemäße Vorrichtung und durch das unter der Vorrichtung liegende Gewebe der untersuchten Körperstelle des Probanden zeigt,

Fig. 4 eine Draufsicht auf ein bevorzugtes kompaktes, multispektrales LED-Modul mit sieben einzeln steuerbaren LED-Halbleitern zeigt und eine entsprechende Positionsmatrix mit den Emissionsmaxima der verwendeten LED-Typen zeigt,

Fig. 5 a) ein integriertes Modul zeigt, bei dem die optische Einheit mit der elektronischen Einheit integriert in einem Gehäuse angeordnet und als gesamte Einheit auf der Haut an der zu beobachtenden Körperstelle des Probanden angebracht ist,

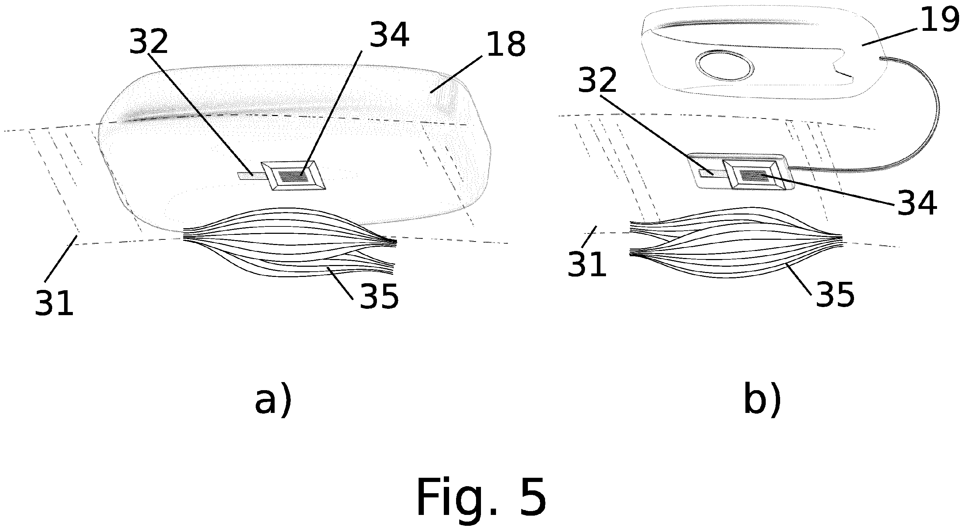

Fig. 5 b) eine Ausführungsform der Vorrichtung mit einem optischen Sensormodul, welches direkt auf der Haut angebracht ist, und mit einer digitalen Datenverarbeitungseinheit, über ein Kabel mit dem optischen Sensormodul verbunden ist und an einer anderen Stelle platziert werden kann,

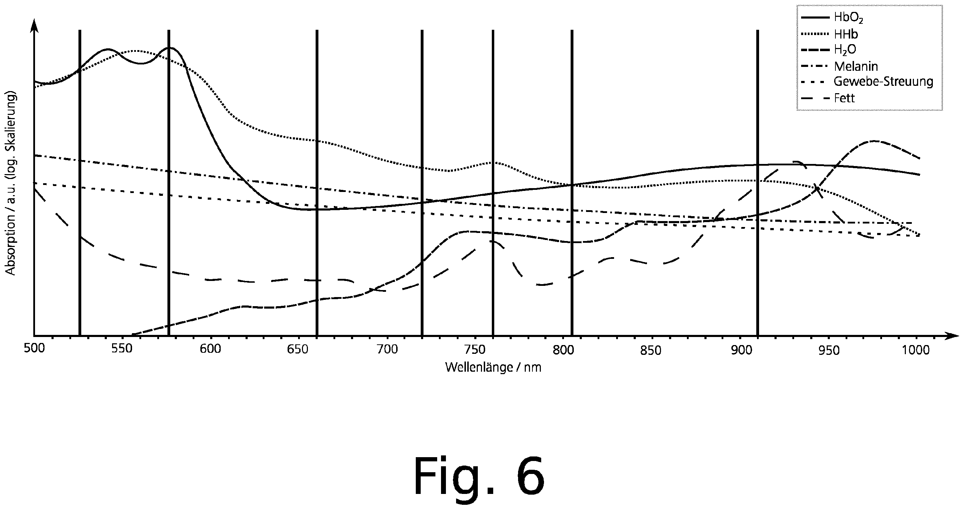

Fig. 6 VIS/NIR-Absorptionsspektren von den für die spektroskopische Beurteilung wichtigen Substanzen des Muskels zeigt, nämlich oxygeniertes Hämoglobin ($HbO_2$), deoxygeniertes Hämoglobin (HHb), Wasser ($H_2O$), Melanin, Fett und die Gewebestreuung, wobei signifikante Wellenlängen markiert sind, die sich gut für eine Datenaufnahme eignen,

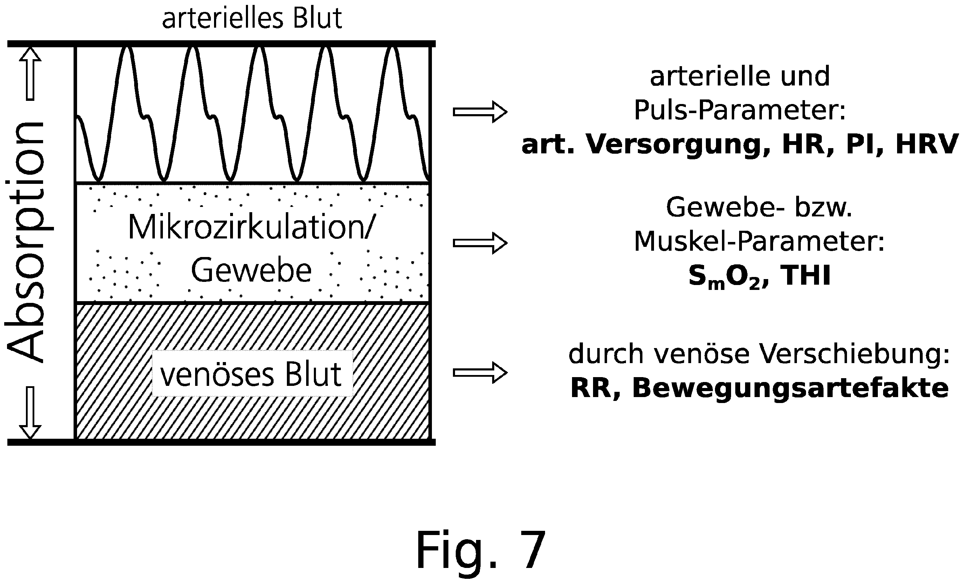

Fig. 7 schematisch konstante und pulsatile Anteile von Körperbestandteilen zeigt, die zur Absorption beitragen,

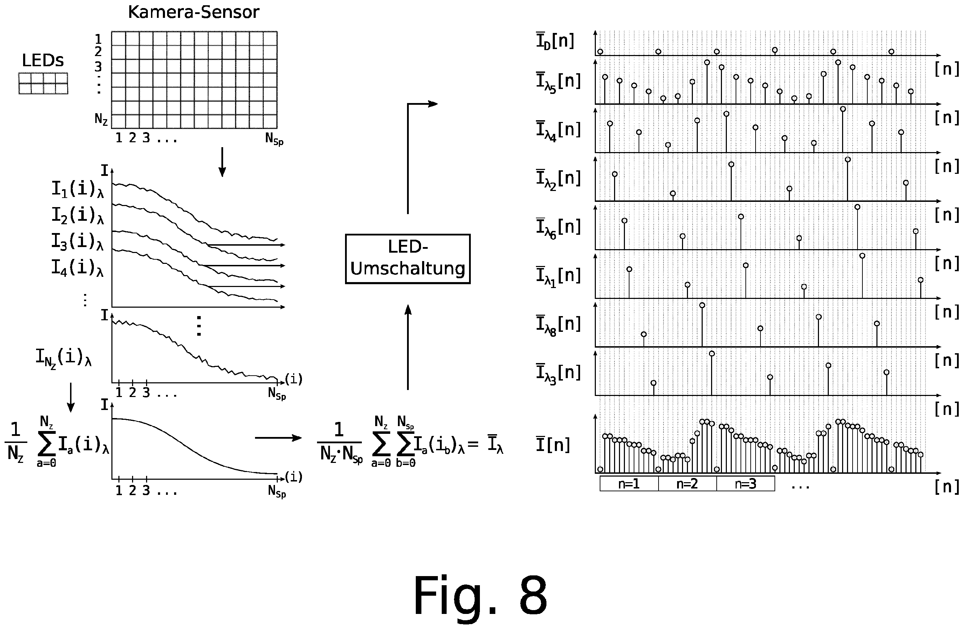

Fig. 8 eine schematische Draufsicht auf eine Beleuchtungseinheit und einen Kamerasensor sowie ausgelesene Intensitätssignale und deren Zusammenfassung zeigt,

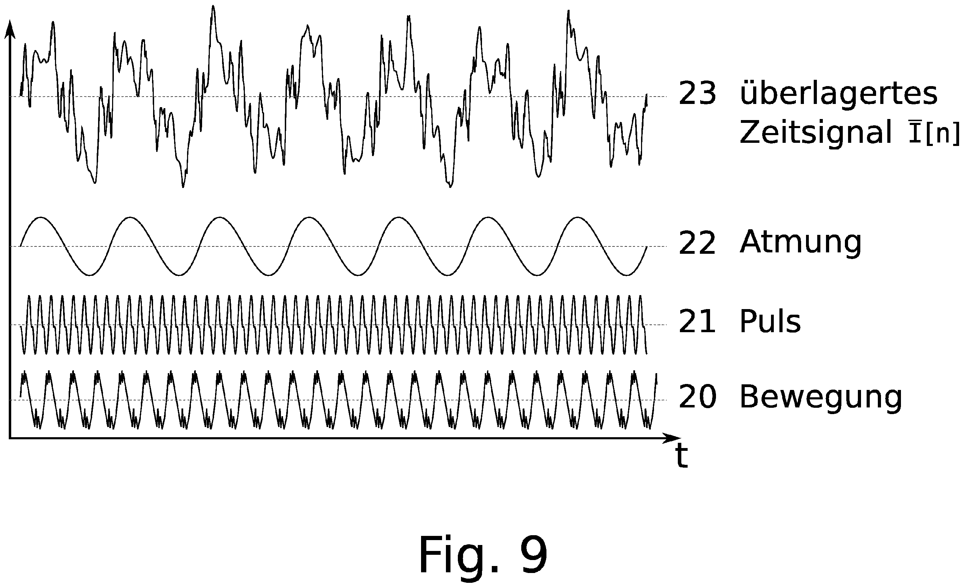

Fig. 9 das wellenlängenübergreifende Kamerasensorintensitätsignal $\bar{I}(\tau)$ als Funktion der Zeit sowie die dazu beitragenden Komponenten zeigt, die aus Atmung, Puls und Bewegung des Probanden resultieren,

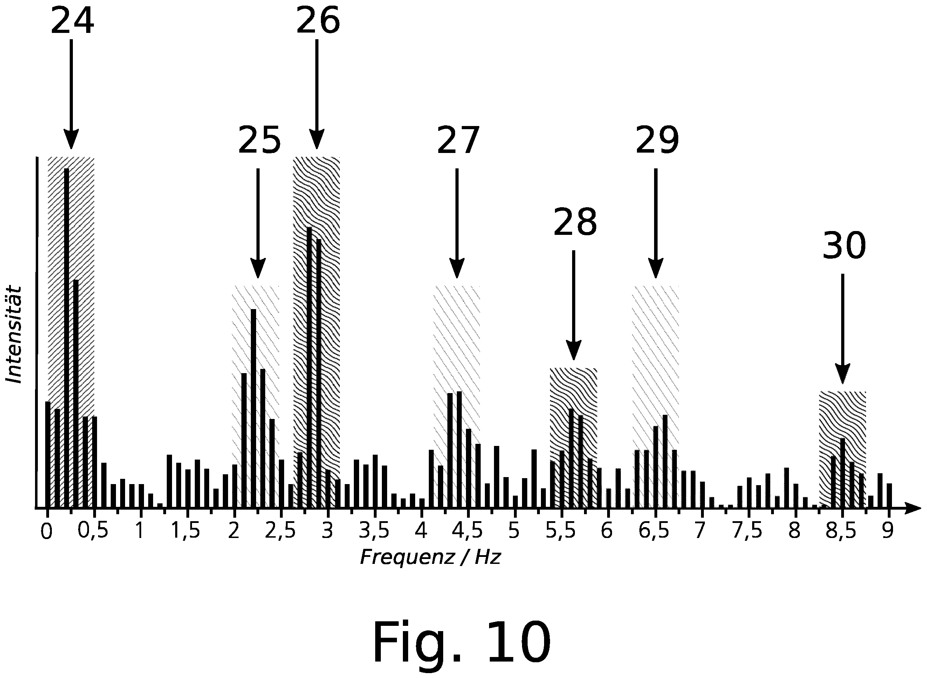

Fig. 10 das Fourier-Spektrum des wellenlängenübergreifenden Kamerasensorintensitätsignals mit darin enthaltenen Verteilungsspitzen, die von Puls, Atmung und Bewegung des Probanden herrühren.

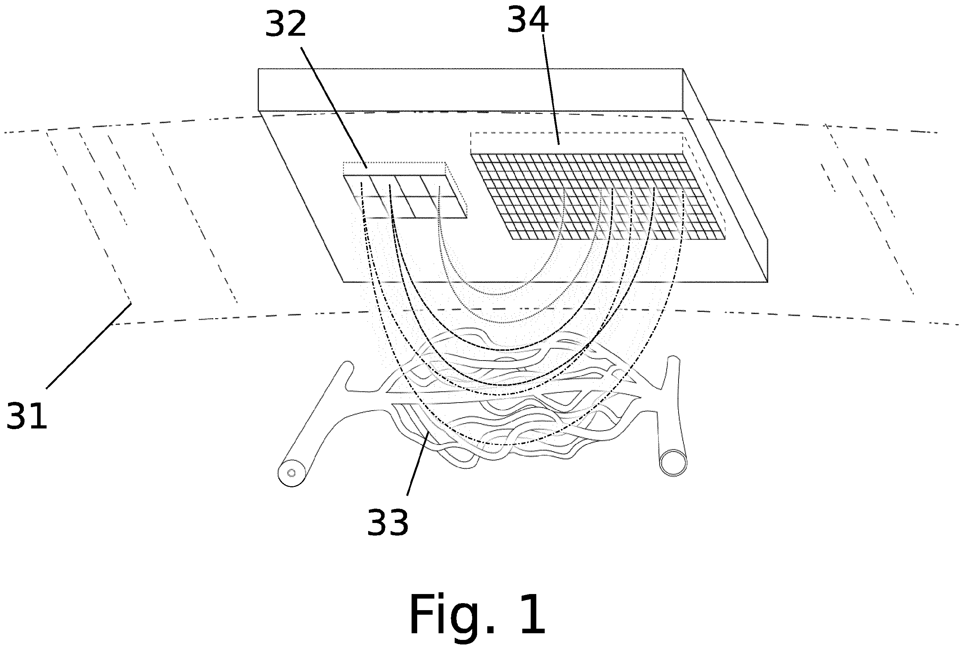

Fig. 1 zeigt als schematische Prinzip-Darstellung den Aufbau einer erfindungsgemäßen Vorrichtung. Die Körperoberfläche, auf der die Vorrichtung angebracht ist, ist mit 31 bezeichnet. Darunter ist das betreffende Messmedium bzw. Gewebe 33 lokalisiert, das zum Beispiel der während des Trainings hauptsächlich belastete Muskel sein kann, grundsätzlich aber auch jede andere Stelle am Körper wie zum Beispiel die Brust, die Schulter oder die Stirn. Auch der Einsatz am Handgelenk kann interessant sein, wobei auch die Integration der Messvorrichtung in ein sogenanntes "Wearable" (z.B. Fitnesstracker oder Smartwatch) denkbar ist.

Die multispektrale Beleuchtungseinheit 32 mit einer Matrix aus LEDs gibt in einem festgelegten Zeitablauf (Aktivierungssequenz) nacheinander Lichtpulse mit Emissionsmaxima bei verschiedenen Wellenlängen in das Gewebe, wobei das eingestrahlte Licht dort nach Eintritt sofort gestreut und in der Ausbreitungsrichtung homogenisiert wird. Bei dem Weg durch das Gewebe wird das Licht von den im Gewebe enthaltenen Substanzen zusätzlich mit festen bekannten Extinktionskoeffizienten absorbiert.

Das Licht tritt an unterschiedlichen Stellen des Gewebes wieder aus. Wichtig ist der Bereich, in dem der optische CMOS-Kamerasensor 34 platziert ist. Dort wird das Licht entfernungsabhängig von der Einstrahlungsstelle gemessen und als analoges Signal aufgenommen. Durch diese Anordnung, bei der die Beleuchtungseinheit 32 und der Kamerasensor 34 in einer Ebene liegen, wird das Licht in der sogenannten Transflexion gemessen, da das Licht zum Teil durch das Gewebe hindurch tritt (Transmission), aber zum Teil auch diffus gestreut bzw. reflektiert wird (Reflexion).

Dieses Signal wird direkt im Kamerasensor 34 verstärkt, digitalisiert und parallel oder seriell für jeden Pixel an eine Prozessoreinheit übertragen.

Bei dem Kamerasensor 34 handelt es sich vorzugsweise um ein hochauflösendes, zweidimensionales CMOS-Digitalkamerasensorarray. Das Array muss ein sogenannter monochromer Sensor sein, also ein Sensor ohne eine strukturierte Farbfiltermatrix. Damit kann der Sensor Licht-Strahlung unterschiedlicher Wellenlängen abhängig von seiner spezifischen Empfindlichkeitskurve aufnehmen.

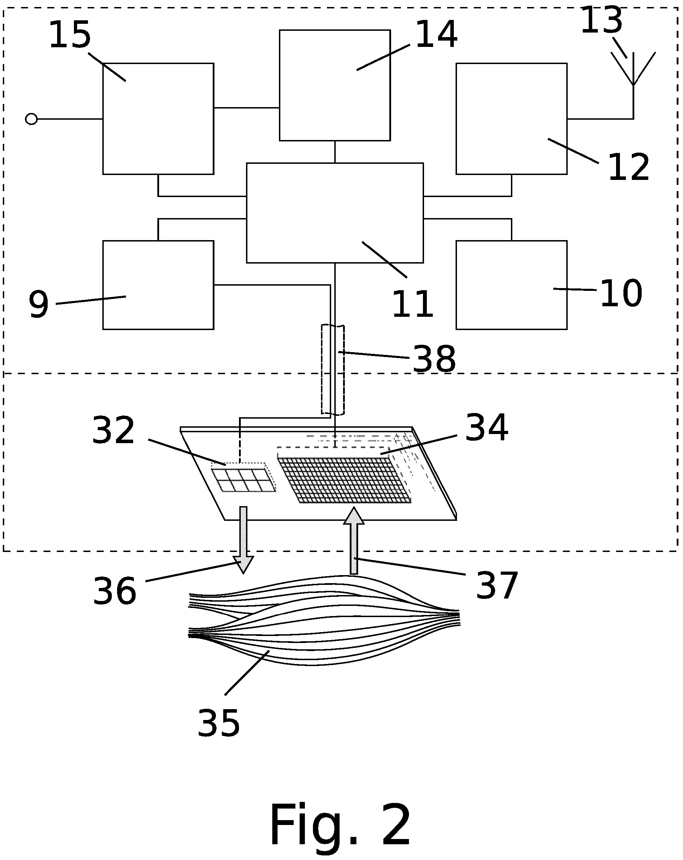

Fig. 2 zeigt ein Blockschaltbild einer bevorzugten Ausführungsform der Vorrichtung. In der Vorrichtung übernimmt eine Datenverarbeitungseinheit (Recheneinheit) 11 die gesamte Steuerung, Datensynchronisation, Datenauslese und -zwischenspeicherung. Die Datenverarbeitungseinheit übernimmt zum einen die Parametrierung des Kamerasensors 34 (unter Parametrierung wird hier das Einstellen der Register des Kamerasensors wie Gain, Skipping, Binning, Belichtungszeit, ROI, Pixelrate etc. verstanden), die digitale Datenaufnahme des Kamerasensors 34 und die Steuerung der multispektralen Beleuchtungseinheit 32. Weiterhin kann die Datenverarbeitungseinheit optional auch eine Datenreduzierung, Datenzwischenspeicherung oder Datenvorverarbeitung durchführen.

Die multispektrale Beleuchtungseinheit 32 strahlt Licht 36 auf den Messbereich 35 des Muskels ab. Dieses Licht tritt wie bereits beschrieben in Transflexion entfernungsabhängig als Analyselicht 37 aus dem Muskel aus und trifft auf den CMOS-Kamerasensor 34. Der Kamerasensor 34 ist ein zweidimensionaler CMOS-Digitalkamera-Sensor. In der einen Richtung (Verbindungsachse von LED-Beleuchtungseinheit 32 und Kamerasensor 34, diese Richtung wird in der vorliegenden Anmeldung als Zeilenrichtung bezeichnet) wird aufgrund der wachsenden Abstände zur Eintrittsstelle in den Muskel die Entfernungsabhängigkeit des austretenden Lichtes erfasst.

In dem Sensor wird bereits die Verstärkung und Digitalisierung der photoelektrischen Signale vorgenommen. Diese werden dann über eine Verbindungsleitung 38 parallel oder seriell an die Datenverarbeitungseinheit 11 übertragen.

Die Intensitätsanpassung zwischen der Beleuchtungseinheit und der Empfindlichkeit des Kamerasensors ist besonders günstig über die Länge der Einschaltzeit der LEDs der Beleuchtungseinheit zu steuern. Damit kann eine sehr präzise Konstantstromquelle 9 die Lichtintensität von einem Lichtpuls zum nächsten sehr stabil halten, die Intensität aber in kleinen Schritten sehr genau eingestellt werden.

Mit Hilfe einer aufladbaren Batterie (Akku) 14 kann das Sensormodul während des Trainings ohne feste Versorgungsverbindung betrieben werden.

Optional können die aktuellen Daten über eine digitale Funkverbindung 12 mit einer in das Gehäuse integrierten Antenne 13 während des Aufnahmeprozess an andere Geräte übermittelt werden. Eine andere Möglichkeit ist die Übertragung der Daten während des Trainings, aber vor allem auch nach dem Abschluss der Datenerfassung kabelgebunden über eine Ausgabeeinheit 15 an andere Geräte. Dieses Kabel kann gleichzeitig zur Stromversorgung und zum Aufladen des Akkus 14 genutzt werden.

In einem Speicher 10 können die aufgenommenen physiologischen Parameterwerte zwischengespeichert werden

Auf anderen Geräten können die Daten weiter aufgearbeitet und ausgewertet werden, sie können aber vor allem angezeigt und in einem größeren Zusammenhang gespeichert werden.

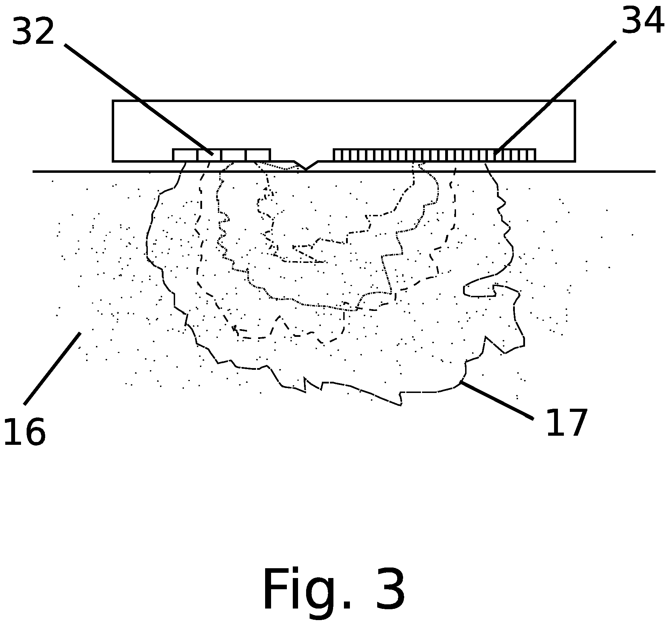

Figur 3 zeigt die optische Anordnung in einem Schnitt durch die Vorrichtung und den darunterliegenden Muskel. So tritt Licht 17 von der jeweils angesteuerten monochromen LED der multispektralen LED-Beleuchtungseinheit 32 in das Gewebe 16 ein und wird in diesem vielfach diffus gestreut und auf dem Weg durchs Gewebe auch abhängig von den Substanzkonzentrationen und der Wellenlänge absorbiert. Da es sich um einen Zufallsprozess handelt, wird das Licht in alle Richtungen gleichverteilt gestreut und reflektiert. Es wurden jedoch nur exemplarisch einzelne Lichtpfade eingezeichnet, die auch auf dem Kamerasensor 34 auftreffen. Wie beschrieben wird dieses Prinzip als Transflexion bezeichnet.

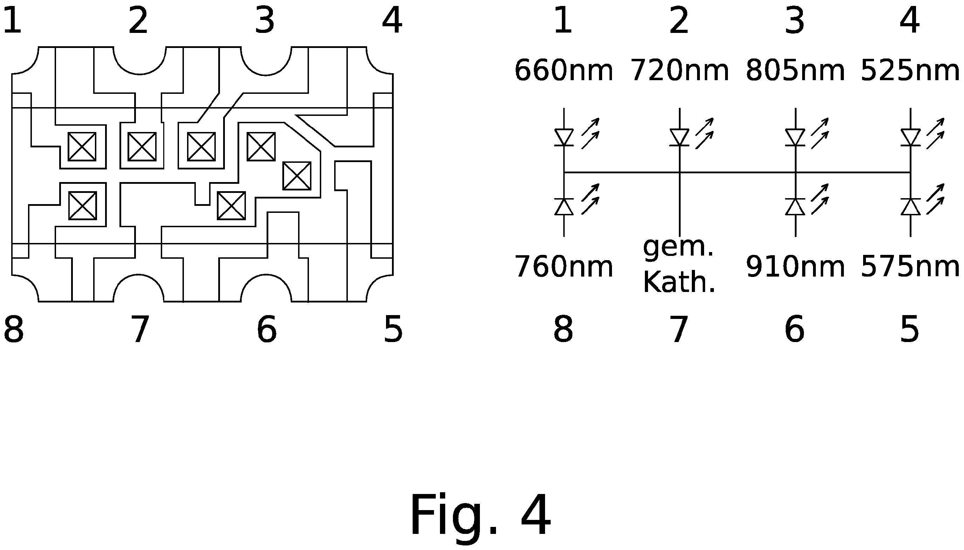

Figur 4 zeigt eine bevorzugte Ausführungsform der multispektralen LED-Beleuchtungseinheit mit sieben separaten Wellenlängen; genauer gesagt haben die LEDs sieben separate Emissionsmaxima, wobei die Halbwertsbreiten der Emissionsspektren etwa 15 nm betragen. Die Größe des Moduls beträgt typisch 4,9 mm x 3,2 mm x 1,2 mm (Länge x Breite x Höhe). Die sieben unterschiedlichen Dyes sind auf einem Träger gemeinsam so angeordnet, dass Sie über eine gemeinsame Kathode separat angesteuert und geschaltet werden können. Die spektrale Zuordnung der LED-Dies ist in der folgenden Tabelle dargestellt, wobei die vorwiegende Funktion des Messsignals bei der Wellenlänge für die physiologische Auswertung stichwortartig angegeben ist.

| | | |
|---|---|---|
| 520 nm | stark pulsatil, aber fast isosbestisch | Pos 4 |
| 575nm | für stark pulsierende $HbO_2$ Bande | Pos 5 |
| 660 nm | für Pulsoximetrie und $SmO_2$ | Pos 1 |
| 720 nm | $SmO_2$ | Pos 2 |
| 760 nm | $SmO_2$ , signifikante Bande im HHb Signal | Pos 8 |
| 805 nm | $SmO_2$, isosbestischer Punkt | Pos 3 |
| 910 nm | Pulsoximetrie und $SmO_2$ | Pos 6 |

Die LED 4 (520 nm) dient zur Unterscheidung der pulsatilen Anteile in Abhängigkeit von der Sauerstoffsättigung. Bei 575 nm (LED 5) ist durch den hohen Absorptionskoeffizienten des $HbO_2$ das Signal durch das oxygenierte Hämoglobin bestimmt, während bei 520 nm die Absorptionskoeffizienten von $HbO_2$ und HHb sehr ähnlich sind (isosbestisch). Somit kann bei einem analytischen Vergleich der zwei Signale zwischen der Zuführung von hochgradig sauerstoffgesättigten Blut in den Arterien und entsättigtem Blut der Mikrozirkulation respektive der Venen unterscheiden.

Die fünf Wellenlängen im NIR Bereich (1, 2, 8, 3, 6) dienen vorwiegend der Datengenerierung für die NIRS Technologie, also der Bestimmung der Muskeloxygenierung. Hierfür ist eine etwas niedrigere Abtastfrequenz ausreichend, da keine höherfrequenten Signale (insbesondere pulsatile Signalanteile) erfasst werden müssen.

Da über die Auswertung der Pulsationen im VIS-Spektralbereich (LED 5 und 4) die Plethkurve bekannt ist, kann im Zusammenhang mit den Signalen der LED 1 und LED 6 die arterielle Sauerstoffsättigung (SpO$_2$) mit der konventionellen Technologie der Pulsoximetrie bestimmt werden.

Die Zuordnung der Wellenlängen der Beleuchtungseinheit zu den Puls-basierten und den Absorptions-basierten Parametern erlaubt es, die einzelnen LEDs mit verschiedenen Taktfrequenzen zu betreiben. Dies wird durch die Festlegung einer Aktivierungssequenz der LEDs erreicht. Eine bevorzugte Ausführungsform einer LED-Aktivierungssequenz ist:

**D, 5,4,2,5, 6,1,5,4,8, 5,3**

Dabei steht D für die Bildaufnahme ohne Aktivierung einer LED (Dunkelbild). Weitere Kriterien für die Sequenz sind, dass LED 5 äquidistant in der Sequenz platziert ist, dass LED 4 nur nach LED 5 folgen darf (wobei dies nicht impliziert, dass auf die 5 die 4 folgen muss) und die LEDs zur Erfassung der Pulsoximetrie-Signale (6,1) direkt aufeinanderfolgend sind. Die LEDs zur Auswertung der NIRS Signale und das Dunkelbild sind zum Auffüllen der Sequenz verteilt, da sie über einen längeren zeitlichen Bereich aufintegriert werden.

Für die Realisierung der Pulsauflösung müssen die dafür vorgesehenen Wellenlängen mit der höchsten Einzelfrequenz aktiviert werden, da wie dargelegt die Bewegungsartefakte und der Puls mit höherer Frequenz abgetastet werden müssen.

Die effektiven Bildraten ergeben sich damit aus der Kamera-Bildrate $f_s$ und dem Vorkommen in der Sequenz zu:

$$f_{D,1,2,3,6,8} = \frac{1}{12} \cdot f_s \qquad ,$$

für die LEDs 1, 2, 3, 6, 8 sowie für das Dunkelbild D,

$$f_4 = \frac{1}{6} \cdot f_s \qquad ,$$

für die LED 4 und

$$f_5 = \frac{1}{3} \cdot f_s \qquad ,$$

für die LED 5.

Wird beispielhaft eine Bildrate von 300fps (frames per second) angenommen, ergeben sich die einzelnen LED-Frequenzen $f_{D,1,2,3,6,8}$ jeweils zu 25 Hz, $f_4$ zu 50 Hz und $f_5$ zu 100 Hz.

In Figur 5 a) ist eine mögliche Ausführungsform dargestellt, bei der die in Figur 2 als Blockschaltbild dargestellte Messvorrichtung vollständig in einem kompakten Gehäuse 18 integriert ist. Das Gehäuse ist so geformt, dass es komfortabel über einem größeren Muskel des Menschen an der Körperoberfläche anzubringen ist und auch in mögliche Befestigungssysteme, die die Vorrichtung auch bei starken Muskelbeanspruchungen stabil an der Oberfläche des Muskels (1) halten, integrierbar ist. Vorteilhaft ist es, wenn das Gehäuse in der Art ausgebildet ist, das es gegen eindringende Feuchtigkeit (Schweiß, Wasser) geschützt ist. Figur 5 b) zeigt eine weitere Ausführungsform, bei der die optische Einheit der Vorrichtung sehr klein gehalten ist und auch direkt am Muskel an der Messstelle befestigt werden kann. Die erweiterte Einheit zur Datenverarbeitung, zur Kommunikation und zur Energieversorgung ist in einem zweiten kompakten Gehäuse 19 untergebracht, welches über ein dünnes Kabel mit der optischen Sensoreinheit verbunden ist. Somit kann die optische Sensoreinheit zum Beispiel fest in ein Kleidungsstück integriert werden und das zweite Gehäuse für Datenaustausch, Energieversorgung und andere Funktionen separat neben der eigentlichen Vorrichtung zur Messwerterfassung angeordnet werden.

Fig. 6 zeigt die Einzelspektren von reinem, oxygeniertem Hämoglobin, von deoxygeniertem Hämoglobin, Wasser, dem Hautpigment Melanin, Fett und von den typischen Streueigenschaften menschlichen Gewebes. Die Auftragung der Absorption in der Y-Achse ist logarithmisch vorgenommen. Die Darstellung soll hauptsächlich die typischen Verläufe der Absorptionskurven und der charakteristischen Absorptionsbanden zeigen. Da die Konzentrationen der einzelnen chemischen Komponenten im Muskel stark variieren können und unbekannte Substanzen zusätzlich zu den Spektren beitragen, ist in der Praxis eine kombinierte Auswertung mit möglichst vielen spektralen Stützpunkten mittels Absorptionsspektren sinnvoll. Dazu wird ein multivariates statistisches Analyseverfahren verwendet, um die Beiträge der Einzelsubstanzen zu den erfassten Spektren zu ermitteln und daraus insbesondere den relativen Beitrag von oxygeniertem Hämoglobin zu deoxygeniertem Hämoglobin (Oxygenierung), die Gewebehämoglobinkonzentration und Konzentrationen der weiteren Substanzen zu bestimmen, insbesondere von Wasser. Anschaulich wird in einem solchen Analyseverfahren das gemessene Spektrum als Linearkombination der Einzelspektren der reinen Substanzen dargestellt und die Koeffizienten der Einzelspektren in derjenigen Linearkombination der Einzelspektren ermittelt, die die beste Übereinstimmung mit dem gemessenen Spektrum liefert. Beispielsweise wird bei der Bestimmung der Konzentration von Wasser die ausgeprägte Absorption von Wasser bei 960 nm herangezogen. Da bei dieser Wellenlänge, wie aus Fig. 6 ersichtlich, auch Absorption durch Fett und durch Hämoglobin auftritt, müssen deren Anteile in dem multivariaten statistischen Analyseverfahren berücksichtigt werden. Dazu wird die Absorption bei 930 nm herangezogen, wo Fett eine hohe Absorption hat, sowie mehrere Stützstellen im Wellenlängenbereich 800-910 nm, die für die Beiträge von oxygeniertem und deoxygeniertem Hämoglobin charakteristisch sind. Bei der kombinierten statistischen Auswertungen der Absorptionen bei allen Wellenlängen können daher die Beiträge der einzelnen Substanzen bestimmt werden, in diesem Beispiel insbesondere der des Wassers.

Mit dem im vorhergehenden im Zusammenhang mit Fig. 4 beschriebene Ausführungsbeispiel mit einer LED-Beleuchtungseinheit mit LEDs auf Positionen 1-6 mit Emissionsmaxima wie in der oben wiedergegebenen Tabelle angegeben ist eine Auswertung und Erfassung der Wasserkonzentration nicht möglich. Wenn die Auswertung auch in Bezug auf Wasser erwünscht ist, müssen wenigstens zwei weitere LEDs vorgesehen sein, wobei eine ein Emissionsmaximum im Bereich von 930 nm, um auf die Absorption von Fett sensitiv zu sein, und eine ein Emissionsmaximum im Bereich von 960 nm, um auf die dort starke Absorption von Wasser sensitiv zu sein.

In Fig. 7 sind schematisch die Anteile der gemessenen Absorption vom Hämoglobin bei der Messung im Muskel dargestellt. Das gemessene optische Signal weist hierbei einen konstanten Anteil und einen pulsatilen Anteil auf.

Der pulsatile Anteil wird durch das Pumpen des arteriellen Blutes vom Herz erzeugt. Die Stärke der Absorption im arteriellen Blut ist bei der Systole und der Diastole nicht gleich. Dies erlaubt eine Differenzierung. Bei einem Herzschlag wird stark sauerstoffgesättigtes Blut in den gemessenen Körperteil gepumpt. Hierbei ist die Sauerstoffsättigung des Hämoglobins bei einem gesunden Menschen im Bereich von 95% bis 99%. Der pulsierende Signalanteil ist wellenlängenabhängig und hängt von der Messstelle und dem erfassten Spektralbereich ab. Aus diesem pulsierenden Signal könnte als Parameter zum Beispiel die Pulsrate (HR), die Pulsratenvariabilität (HRV) und der Pulsationsindex (PI) bestimmt werden.

Das vom Gewebe stammende Signal gliedert sich in zwei Bereiche. Der eine Anteil ist von den Inhaltsstoffen des Gewebes abhängig, der andere Anteil hängt von den Streueigenschaften des Gewebes ab, welche die realen Lichtwege beeinflussen. Aus dem Bereich der Inhaltsstoffe lassen sich die Muskeloxygenierung, der Gewebehämoglobinindex, im Prinzip aber auch der Fett- und Glukoseanteil (Energieversorgung) berechnen. Die Streueigenschaften des Gewebes können über die entfernungsabhängige Auswertung auf dem CMOS Sensorarray erfasst werden.

Über die Verschiebung des venösen Blutanteils können zum einen die Atemfrequenz, zum anderen die Bewegungen des Sportlers erfasst werden. Während eines Atemzyklus wird eine größere Blutmenge aus dem Zentralbereichs des Körpers in die peripheren Bereiche und wieder zurück verschoben.

Figur 8 zeigt schematisch die Generierung der verschiedenen Signale für die Berechnung der unterschiedlichen Parameter. Für die Einzelbilder der Kamera werden die räumlichen Dimensionen $N_{sp}$ als Spaltenanzahl und $N_z$ als Zeilenanzahl ausgewertet, wobei die Zeilenrichtung (mit der Spaltennummer $i$ als Laufvariable) parallel zur Verbindungsachse von LED-Beleuchtungseinheit und Kamerasensor und die Spaltenrichtung orthogonal dazu verläuft. Im ersten Schritt werden die einzelnen Zeilen, deren Intensität mit steigender Spaltenzahl $i$ und damit steigender Entfernung von der Lichtquelle abnimmt, miteinander gemittelt. Das über alle Zeilen gemittelte Ergebnis stellt eine Intensitätsfunktion in Zeilenrichtung $i$ dar, mit $i$ = 1, 2 ... $N_{sp}$:

$$\bar{I}(i)_{\lambda m} = \frac{1}{N_Z} \sum_{a=0}^{N_Z} I_a(i)_{\lambda m}$$

Da für jedes Bild innerhalb der Aktivierungssequenz die Wellenlänge verschieden ist, wird der Index $\lambda m$ mitgeführt. Da für jede Aktivierung innerhalb der Aktivierungssequenz ein Aktivierungsstartzeitpunkt gegeben ist stellt die gemittelte Intensität $\bar{I}(i)_{\lambda m}$ auch eine Zeitreihe als Funktion der Zeit $\tau$ dar, die weiter unten angegeben ist. Den einzelnen Aktivierungsphasen innerhalb der Aktivierungssequenz sind jeweils ein Aktivierungsstartzeitpunkt $t_k$ mit $k$ = 1,2,...$M$ (mit $M$ als Anzahl der Aktivierungsphasen innerhalb einer Sequenz) und eine Leuchtdauer zugeordnet, wobei beachtet werden muss, dass die Leuchtdauer pro LED-Typ individuell verschieden sein kann, aber natürlich immer kleiner als die Zeitdauer zwischen zwei aufeinanderfolgenden Startzeitpunkten $t_{k-1}$ und $t_k$ sein muss.

Die Zeitdauer $T_A$ zwischen den einzelnen Aktivierungsstartzeitpunkten ist durch die Kamera-Bildrate $f_A$ vorgeben, so dass die Aktivierungsstartzeitpunkte der LEDs äquidistant zueinander angeordnet sind, die Ausschaltzeitpunkte jedoch individuell verschieden sein können:

$$T_A = \frac{1}{f_A} = t_k - t_{k-1} = const.$$

Die Zeitdauer $T_S$ zwischen zwei Startzeitpunkten aufeinanderfolgender Aktivierungssequenzen ergibt sich wiederum aus der Zeitdauer $T_A$ zwischen den einzelnen Aktivierungsstartzeitpunkten innerhalb der Aktivierungssequenz und der Anzahl $M$ der Aktivierungen der Aktivierungssequenz, wobei davon ausgegangen wird, dass die Aktivierungssequenzen stetig ohne Pause wiederholt werden:

$$T_S = \frac{1}{f_S} = M \cdot T_A = M \cdot (t_k - t_{k-1}) = const.$$

Die einzelnen Zeitpunkte, an denen jeweils die gleiche LED angeschaltet ist, ergeben das Signal $\overline{I}_{\lambda m}[n,t_k]$ als Zeitreihe, mit $\lambda m$ als Index für die Emissionsmaxima der LED-Typen **D,1,2,3,4,5,6,8,** wobei D kein LED-Typ im eigentlichen Sinne ist, sondern die Abwesenheit von Beleuchtung (Dunkelphase) darstellt. Statt der laufenden Nummer n der Aktivierungssequenz und dem Aktivierungsstartzeitpunkt $t_k$ innerhalb der Aktivierungssequenz kann auch die absolute Zeit $\tau(n,tk)$ des jeweiligen Aktivierungsstartzeitpunkts angegeben werden $\tau(n, t_k) = n \cdot T_s + t_k$. Die entsprechenden Signale (z.B. $\overline{I}[\tau]$ und $\overline{I}[n]$) können daher als äquivalent angesehen werden.

Die gemittelten Intensitätsverteilungen $\overline{I}(i)_{\lambda m}$ der unterschiedlichen Wellenlängen dienen teilweise bereits als Eingangsdaten für die Berechnung der spektralen Absorptions-basierten Parameter (z.B. $SmO_2$). Zur Erhebung der pulsatilen (Puls-basierten) Parameter wird die gemittelte Intensitätsverteilung $\overline{I}(i)_{\lambda m}$ über die Spalten $i$ gemittelt. Damit wird die Intensitätsverteilung $\overline{I}(i)_{\lambda m}$ zum Intensitätswert $\overline{I}_{\lambda m}$, wobei jedem dieser Intensitätswerte eine Zeit $\tau(n,t_k) = n \cdot T_s + t_k$ der jeweiligen Aktivierung zugeordnet ist, die in der folgenden Formel nicht mit aufgeführt ist:

$$\overline{I}_{\lambda m} = \frac{1}{N_{Sp}} \sum_{i=0}^{N_{Sp}} \overline{I}(i)_\lambda = \frac{1}{N_Z \cdot N_{Sp}} \sum_{a=0}^{N_Z} \sum_{i=0}^{N_{Sp}} I_a(i)_{\lambda m}$$

Tatsächlich handelt es sich hierbei für jede Wellenlänge Am um eine Zeitreihe $\overline{I}_{\lambda m}(\tau)$, wie auf der rechten Seite in Fig. 8 dargestellt.

Wird die LED in der bereits beschriebenen Aktivierungssequenz D,5,4,2,5,6,1,5,4,8,5,3 beschaltet, ergeben sich zunächst die acht untereinander dargestellten Zeitreihen $\overline{I}_D(\tau), \overline{I}_5(\tau), \overline{I}_4(\tau) ... \overline{I}_3(\tau)$ Diese können auch in einer Zeitreihe $\overline{I}[\tau]$ zusammengefasst werden, wie in Fig. 8 rechts ganz unten dargestellt.

Die jeweiligen Signale $\overline{I}(i)_{\lambda m}, \overline{I}_{\lambda m}[\tau]$ und $\overline{I}[\tau]$ können bei der Auswertung separat, aber auch in Kombination miteinander genutzt werden.

In der vorliegenden Erfindung wird diese Kombination genutzt, um die Auswertung der einzelnen Parameter auch bei intensiver Bewegung, die zudem durch starke Regelmäßigkeit gekennzeichnet ist, zu ermöglichen.

In Figur 9 sind die zum Signal $\overline{I}[n]$ beitragenden Komponenten dargestellt. Das durch die Muskelkontraktion und Erschütterung (Bewegung) entstehende periodische Signal (**20**) wird eine Verschiebung des im Muskelgewebe befindlichen Hämoglobins verursachen. Das vom Herzschlag verursachte pulsierende Signal (**21**) wird sich wie bereits dargestellt hauptsächlich auf das oxygenierte arterielle Blut auswirken und ist zusätzlich durch seine markante Form durch Diastole, Systole und Dikrotie (durch Aortenklappenschluss) des Herz-Zyklus gekennzeichnet. Die durch die Atmung verursachte Frequenz (**22**) ist durch eine Verschiebung hauptsächlich des venösen Anteils (hoher HHb Anteil) gekennzeichnet. Die Atemfrequenz kann gerade bei dem Einsatz der sportlichen Leistungsuntersuchung immer niedriger als die Pulsfrequenz angenommen werden.

Für die drei dargestellten Einflüsse Puls, Atmung und (Muskel-)Bewegung gelten folgende Eigenschaften, die durch die vorliegende Erfindung genutzt werden um eine möglichst präzise Separation zu ermöglichen.

    1. Das Pulssignal ist gekennzeichnet durch:

    1.1. eine charakteristische Form, die durch Systole und Diastole des Herzens sowie der dem Schließen der Aortenklappe und der flexiblen Beschaffenheit der Arterien verursacht wird und durch Fourier-Transformation ein spezifisches Frequenz-Spektrum mit mehreren harmonischen Oberwellen besitzt,

1.2. eine Oxygenierungserhöhung pro Puls, da das Signal durch arterielle Pulswelle verursacht wird (art. Sättigung SpO2 typisch bei 98%),

1.3. Gradienten/Änderungen der Rate in einem definierten Zeitbereich (Herzraten-Variabilität) sind physiologisch begrenzt.

2. Das Atmungs-Signal ist gekennzeichnet durch:

2.1. Einen nahezu sinusförmigen Verlauf,

2.2. eine geringere Frequenz als das Pulssignal,

2.3. da das Signal durch eine hauptsächlich venöse Blutverschiebung verursacht wird:

2.3.1. keine Oxygenierungserhöhung pro Puls

2.3.2. im NIR-Bereich relative Signaländerung, da sich durch erhöhte Eindringtiefe das Verhältnis von konstantem (Venen/Mikrozirkulation) zu variablen Anteil (Arterien) ändert

2.4. Gradienten der Rate (Atemfrequenz-Variabilität) sind physiologisch begrenzt.

3. Das Bewegungs-Signal ist gekennzeichnet durch:

3.1. einen periodischen, aber nicht Sinus-förmigen Verlauf mit vielen (ungeordneten/undefinierten) Oberwellen,

3.2. da das Signal hauptsächlich durch venöse/mikrozirkuläre Blutverschiebung verursacht wird:

3.3. keine Oxygenierungserhöhung pro Puls
3.3.1. im NIR-Bereich relative Signaländerung, da sich durch erhöhte Eindringtiefe das Verhältnis von konstantem (Venen/Mikrozirkulation) zu variablen Anteil (Arterien) ändert.

3.4. Für verschiedene Sportarten können Bewegungsmuster bestimmt werden und durch angepasste Filterung (bspw. Wavelet-Analyse mit speziellen Wavelets) und Vorhersage-Modellen (z.B. ARMA-Modell; Autoregressive-Moving Average) extrahiert werden.

Mit diesem Wissen und der Ausgestaltung der Erfindung (Auswahl und Anordnung der LED-Wellenlängen, Einsatz eines schnellen 2D-Kamerasensors) können alle interessierenden physiologischen Parameter bestimmt werden, und zwar sowohl solche die mit dem Puls zusammenhängen, als auch solche, die mit der Atmung in Zusammenhang stehen, als auch solche, die mit der Oxygenierung des Gewebes zusammenhängen. Dadurch können alle interessierenden physiologischen Parameter des Probanden mit einer einzigen Sensorvorrichtung erfasst werden, die zudem so kompakt ausgeführt werden kann, dass sie auf eine Stelle des Körpers des Probanden gerichtet angebracht werden kann, ohne dabei die sportliche Betätigung des Probanden zu behindern. Mit im Stand der Technik waren für die verschiedenen Gebiete (Puls, Atmung, Oxygenierung der Muskeln) verschiedene Sensoren oder Messvorrichtungen notwendig, die dann an verschiedenen Orten des Körpers des Probanden angebracht werden mussten. Mit der vorliegenden Erfindung kann mit einem einzigen kompakten optischen Sensor der gesamte physiologische Parametersatz ohne Behinderung der sportlichen Betätigung erfasst werden.
Die beschriebenen Eigenschaften der Signale Puls, Atmung und Bewegung können wie folgt ausgewertet werden. Die Nummerierung der Eigenschaften wird im Folgenden aufgenommen und zur besseren Darstellung genutzt.

1. Bestimmung des Pulssignals:

1.1. Charakteristische Form der Pulswelle:

In jedem Pulszyklus wird dem Gefäßsystem hoch oxygeniertes Blut zugeführt, das aufgrund des Widerstands der kleineren Gefäße nicht sofort abfließen kann. Dadurch entsteht ein Maximum in der Blutdruckkurve, der dem systolischen Blutdruck entspricht. Durch den Schluss der Aortenklappe entsteht in der Blutdruckkurve eine Inzisur. Der Blutdruck fällt durch das Abfließen des Blutes in die Peripherie und sinkt bis zum nächsten Herzschlag auf ein Minimum, dem sogenannten diastolischen Blutdruck. Der Verlauf der Blutdruckkurve, die sich aus Systole, Inzisur und Diastole zusammensetzt, wird durch die Flexibilität der Gefäße mit zunehmender Entfernung vom Herzen "Tiefpass-gefiltert" und die charakteristische dikrotische Pulswelle entsteht.

Diese Charakteristik der Pulswelle kann genutzt werden um durch Analyse des zeitlichen Verlaufs $\overline{I}[n]$ die Bestimmung der Puls-Frequenz zu ermöglichen. Da die Pulswelle nicht sinusförmig verläuft, entstehen durch die Transformation des Zeitsignals in den Frequenzraum (durch Fourier-Transformation) neben der Grundfrequenz weitere Oberfrequenzen, die als ganzzahlige Vielfache der Grundfrequenz ausgebildet sind. Dies ist in Fig. 10 für die Verteilungsspitze (Peak) 25 gegeben, die der Grundfrequenz des Pulses entspricht und zu der Oberwellen 27 (bei der doppelten Frequenz) und 29 (bei der dreifachen Grundfrequenz) identifizierbar sind. Damit kann auch im Frequenzraum das Pulssignal von der Atmung abgegrenzt werden, da diese einem Sinus ähnlich ist und ohne oder mit kaum ausgeprägten Oberwellen auftritt. Dies ist in Fig. 10 für die Verteilungsspitze 24 der Fall, die aufgrund dessen mit der Atmung assoziiert werden kann.

1.2. Oxygenierungserhöhung pro Puls:

Zur Prüfung der Oxygenierungsänderungen pro Puls werden die Signale $\overline{I}_{\lambda 5}[n]$, sowie $\overline{I}_{\lambda 4}[n]$ ausgewertet. Das Signal $\overline{I}_{\lambda 4}[n]$ bei 525 nm dient als Bezugsgröße, da hier ein isosbestischer Punkt abgetastet wird, dessen Intensität nicht von der Oxygenierung abhängt. Wird dazu das Signal bei 575 nm $\overline{I}_{\lambda 5}[n]$ hinzugezogen, das durch einen großen Absorptions-Abstand zwischen oxygeniertem und deoxygeniertem Hämoglobin gekennzeichnet ist, lässt sich die Sauerstoff-Sättigung für jeden einzelnen Puls auswerten und so eine Trennung zwischen Signalen aufgrund des Pulses und aufgrund von Bewegung erreicht werden.

1.3. Begrenzte Änderung der Pulsrate:

Zusätzlich kann durch physiologisch bedingte Grenzen der Änderung der Herzrate (Herzraten-Variabilität) die Pulswelle durch ein angepasstes dynamisches Filter (Bandpassfilter im Frequenzbereich) mit der Herzrate als (dynamische) Mittenfrequenz und der zugelassenen Variabilität als Bandbreite so gefiltert werden, dass die Atmung oder die Bewegung nicht einbezogen werden, sofern die entsprechenden Raten (bzw. Frequenzen) hier nicht in das definierte Frequenz-Fenster hineinlaufen. Dies wird für die Atemfrequenz nie der Fall sein, da das Pulssignal immer deutlich höhere Frequenz aufweist als das Atmungs-Signal. Die Trennung des arteriellen Pulssignals und der Bewegung wird in diesem Fall durch die Prüfung der Oxygenierung erreicht.

2. Detektion der Atmung:

2.1, 2.2 und 2.4: Sinusförmig, niedrigere Frequenz als der Puls und begrenzte Änderung der Atemfrequenz:

Das Prinzip des dynamischen Filters kann auch auf das Signal der Atmung angewendet werden. Hier wird ein Frequenz-Spektrum erwartet, das nur einen signifikanten Peak aufweist, da das Signal der Atmung sinus-ähnlich verläuft und keine oder kaum ausgeprägte Oberwellen besitzt (Eigenschaft 2.1), was in dem Frequenzspektrum in Fig. 10 für die Verteilungsspitze 24 der Fall ist. Das Filter kann unter der Annahme parametrisiert werden, dass die Atemfrequenz deutlich niedriger als die Puls-Frequenz ist (Eigenschaft 2.2) und die Variabilität der Atem-Frequenz in einem definierten Zeitfenster deutlich eingeschränkt ist (Eigenschaft 2.4).

2.3. Durch venöse Blutverschiebung:

2.3.1. Geringe Oxygenierung pro Puls:

Durch Auswertung der Oxygenierung mit den Signalen $\overline{I}_{\lambda 4}[n]$ und $\overline{I}_{\lambda 5}[n]$ kann eine Abgrenzung des (arteriellen) Pulssignals und des Atemsignals erreicht werden. Dadurch kann eine Entscheidung abgesichert werden, ob eine gefundene Frequenz der Atmung oder dem Puls zuzuordnen ist.

2.3.2. Relative Signaländerung im NIR-Bereich:

Dabei ist weiterhin zu beachten, dass die venöse Verschiebung eine Änderung des Signals im NIR-Bereich zur Folge hat, da im NIR-Bereich relativ betrachtet höhere Signal-Schwankungen erzeugt werden als im VIS-Bereich. Dies wird verursacht, da im NIR-Bereich höhere Eindringtiefen ins Gewebe erreicht werden und sich damit der Anteil der venösen bzw. mikrozirkulären Signale gegenüber den arteriellen Signalen verändert.

3. Bewegung:

Das Bewegungssignal enthält zwar keine Informationen aus denen direkt die physiologischen Leistungsparameter errechnet werden, jedoch muss für eine gefundene signifikante Frequenz entschieden werden, ob dies der Puls, die Atmung oder durch die Bewegung verursacht wurde. Die Prüfung der Bewegungssignale erfolgt daher zur Absicherung der Puls- und Atmungs-Erkennung und dem Ausschluss von rhythmischen Bewegungen. Dies kann wieder durch die Prüfung der Oxygenierung (Eigenschaft 3.2.1) und der Änderung der Signalanteile im NIR-Bereich (Eigenschaft 3.2.2) erreicht werden.

Zusätzlich zu der Bestimmung der Oxygenierung können zur Trennung der Bewegungssignale die Eigenschaften des zeitlichen Verlaufs während der sportlichen Betätigung genutzt werden. Die Signale, die durch die Muskelkontraktion bzw. der Erschütterung der Messvorrichtung erzeugt werden, sind durch eine Periodizität gekennzeichnet, die sich je nach durchgeführter Sportart unterscheiden kann (Eigenschaft 3.1). So wird für die Durchführung eines Lauftrainings ein stärkerer Einfluss von Erschütterungen erwartet, als bei einem Training mit dem Fahrrad. Die unterschiedlichen Betätigungen erzeugen spezifische Signalformen, die den Einsatz von angepassten Analyseformen wie z.B. der Wavelet-Analyse ermöglichen. Dabei kann das Bewegungsmuster als Wavelet hinterlegt werden und das Signal damit untersucht werden. Das Wavelet kann als spezielles Bandpassfilter angesehen werden, dessen Verschiebung im Frequenzbereich dort ein Maximum erzeugt, wo die Frequenz der Bewegung auftritt.

In Figur 10 ist das Frequenz-Spektrum dargestellt, das aus dem diskreten Zeitsignal $\overline{I}[n]$ erzeugt werden kann. Die Atmung 24 ist durch einen einzelnen Peak gekennzeichnet, der Puls wird durch die Grundfrequenz 25 und die Oberwellen 27 und 29 beschrieben. Das Signal der Bewegung besitzt die Grundwelle 26 sowie Oberwellen 28 und 30. Daraus ist ersichtlich, dass die Prüfung der Puls-frequenz durch die Abfrage der Oberwellen nicht zwangsläufig ausreicht, um entscheiden zu können, ob es sich bei einer der Grundfrequenzen 25 und 26 um Puls oder Bewegung handelt. Dafür kann die Abfrage der Oxygenierung durchgeführt werden.

**Patentansprüche**

1. Vorrichtung zur kontinuierlichen und nichtinvasiven Bestimmung von physiologischen Parametern eines Probanden bei körperlicher Belastung mit

   einer zur Auflage auf die Haut des Probanden an einer gewünschten Messstelle ausgestaltete Beleuchtungseinheit (32) auf LED-Basis mit einer Mehrzahl von unterschiedlichen, nebeneinander angeordneten LED-Typen, deren Emissionsmaxima bei verschiedenen Wellenlängen $\lambda 1, \lambda 2 \ldots \lambda L$ vom sichtbaren bis in den NIR-Wellenlängenbereich liegen, wobei die Beleuchtungseinheit (32) wenigstens einen LED-Typ mit einem Emissionsmaximum unterhalb von 590 nm umfasst,

   einem Fotosensor, der zur Auflage auf die Haut des Probanden ausgestaltet ist, um von der Beleuchtungseinheit (32) ausgesendetes und durch den Körper des Probanden zu einem Austrittsort im Bereich des Fotosensors gelangendes Licht zu erfassen,

   einer Datenverarbeitungseinheit (11), die zum Auslesen des Fotosensors mit diesem verbunden ist und die mit der Beleuchtungseinheit (32) in Verbindung steht und dazu eingerichtet ist, die unterschiedlichen LED-Typen jeweils einzeln in einer vorgegebenen Aktivierungssequenz zu aufeinanderfolgenden Aktivierungsstartzeitpunkten $t_k$ (k=1, 2 ... M) für eine jeweils vorgegebene Aktivierungsdauer zu aktivieren und die Aktivierungssequenz mit einer Taktfrequenz als Folge n= 1, 2 ... N von Aktivierungssequenzen zu wiederholen, wobei die Taktfrequenz ausreichend hoch zur Auflösung des Pulses des Kreislaufs des Probanden ist,

   **dadurch gekennzeichnet, dass**

   als Fotosensor ein Kamerasensor (34) auf CCD- oder CMOS-Basis mit einem zweidimensionalen Array mit Zeilen und Spalten von Sensorelementen eingesetzt ist,

   der Kamerasensor (34) so zur Beleuchtungseinheit (32) angeordnet ist, dass er auf der Haut auf dergleichen Seite des Körperteils wie die Beleuchtungseinheit und benachbart zu dieser zur Auflage kommen kann, um durch Transflexion durch den Körper des Probanden zum Kamerasensor gelangendes Licht erfassen zu können,

   die Datenverarbeitungseinheit (11) dazu eingerichtet ist, den Kamerasensor in jeder Aktivierungssequenz zu den Aktivierungsstartzeitpunkten $t_k$ (k=1, 2 ... M) und über die jeweilige Aktivierungsdauer auszulesen, und die erfassten Intensitäten der Sensorelemente über Unterbereiche von Sensorelementen zusammengefasst und der jeweiligen Aktivierungssequenz aus der Folge n=1, 2 ... N von Aktivierungssequenzen und dem jeweiligen Aktivierungsstartzeitpunkt $t_k$ (k=1, 2 ... M) zugeordnet als Zeitreihen aufzuzeichnen, wobei Unterbereiche des Kamerasensors zusammengefasst werden, indem Zeilen des Kamerasensors ausgelesen werden, die parallel zur Verbindungsachse zwischen Beleuchtungseinheit und Kamerasensor liegen und entlang derer die Entfernung von der Beleuchtungseinheit zunimmt, und parallele Zeilen zu einer einzigen gemittelten Zeile zusammengefasst werden, und die Datenverarbeitungseinrichtung dazu eingerichtet ist, die gemittelte Zeile als Funktion des Abstands von der Beleuchtungseinheit zur Muskeloximetrie auszuwerten.

2. Vorrichtung nach Anspruch 1, **dadurch gekennzeichnet, dass** die Datenverarbeitungseinheit (11) dazu eingerichtet ist, jeweils Zeilen des Kamerasensors (34), die parallel zur Verbindungslinie zwischen Beleuchtungseinheit (32) und Kamerasensor (34) verlaufen, auszulesen und zur Zusammenfassung von Unterbereichen von Sensorelementen alle Zeilen zu einer gemittelten Zeilenintensität

$$\bar{I}(i)_{\lambda m}(n, t_k) = \frac{1}{N_Z} \sum_{a=0}^{N_Z} I_a(i)_{\lambda m}(n, t_k)$$

zu mitteln, wobei i=1,2 ... $N_{Sp}$ der durchlaufende Spaltenindex und $N_{Sp}$ die Anzahl der Spalten des Kamerasensors ist und der Index Am die Wellenlänge des Emissionsmaximums des jeweiligen LED-Typs symbolisiert und als gemittelte Intensität $\bar{I}(i)_{\lambda m}(n, t_k)$ der jeweiligen Aktivierungssequenz n aus der Folge n=1, 2 ... N von Aktivierungssequenzen und dem jeweiligen Aktivierungsstartzeitpunkt $t_k$ (k=1, 2 ... M) zugeordnet als Zeitreihen aufzuzeichnen.

3. Vorrichtung nach Anspruch 1 oder 2, **dadurch gekennzeichnet, dass** die Datenverarbeitungseinheit (11) dazu eingerichtet ist, die Intensitäten der Sensorelemente über alle Zeilen und Spalten zu einer über den Kamerasensor (34) integrierten Kamerasensorintensität

$$\bar{I}_{\lambda m} = \frac{1}{N_Z \cdot N_{Sp}} \sum_{a=0}^{N_Z} \sum_{i=0}^{N_{Sp}} I_a(i)_{\lambda m}$$

zusammenzufassen, wobei i=1,2 ... $N_{Sp}$ der durchlaufende Spaltenindex, $N_{Sp}$ die Anzahl der Spalten des Kamerasensors, a der durchlaufende Zeilenindex, $N_z$ die Anzahl der Zeilen des Kamerasensors und der Index Am die Wellenlänge des Emissionsmaximums des jeweiligen LED-Typs symbolisiert, und diese der jeweiligen Aktivierungssequenz n aus der Folge n=1, 2 ... N von Aktivierungssequenzen und dem jeweiligen Aktivierungsstartzeitpunkt $t_k$ (k=1, 2 ... M) zugeordnet als Zeitreihen $\bar{I}_{\lambda m}$(n, $t_k$) der Kamerasensorintensität aufzuzeichnen.

4. Vorrichtung nach Anspruch 3, **dadurch gekennzeichnet, dass** die Datenverarbeitungseinheit (11) dazu eingerichtet ist, die Zeitreihen $\bar{I}_{\lambda m}$(n, $t_k$) der Kamerasensorintensität der verschiedenen Wellenlängen $\lambda 1$, $\lambda 2$ ... $\lambda L$ zu einer wellenlängenübergreifenden einzelnen Zeitreihe $\bar{I}$(n, $t_k$) der Kamerasensorintensität zusammenzufassen und aufzuzeichnen.

5. Vorrichtung nach einem der vorhergehenden Ansprüche, **dadurch gekennzeichnet, dass** die Beleuchtungseinheit (32) wenigstens einen LED-Typ mit Emissionsmaximum im Bereich von 500 nm bis 540 nm und eine LED-Typ mit Emissionsmaximum im Bereich von 570 nm bis 585 nm umfasst.

6. Vorrichtung nach Anspruch 5, **dadurch gekennzeichnet, dass** die Beleuchtungseinheit (32) im NIR-Wellenlängenbereich wenigstens drei unterschiedliche LED-Typen umfasst, deren Emissionsmaxima einen NIR-Wellenlängenbereich von 600 nm bis 1.100 nm aufspannen.

7. Vorrichtung nach Anspruch 6, **dadurch gekennzeichnet, dass** die Beleuchtungseinheit (32) in dem aufgespannten NIR-Wellenlängenbereich wenigstens vier unterschiedliche LED-Typen mit Emissionsmaxima verteilt in dem aufgespannten NIR-Wellenlängenbereich umfasst.

8. Vorrichtung nach einem der vorhergehenden Ansprüche, **dadurch gekennzeichnet, dass** die Beleuchtungseinheit (32) im NIR-Wellenlängenbereich eine Mehrzahl von LED-Typen mit Emissionsmaxima verteilt in dem Wellenlängenbereich 800-1100 nm aufweist, einschließlich eines LED-Typs mit Emissionsmaximum bei 960 nm, eines LED-Typs mit Emissionsmaximum bei 930 nm und wenigstens zweier LED-Typen mit Emissionsmaxima im Bereich von 790-920 nm, und dass die Datenverarbeitungseinheit (11) dazu eingerichtet ist, aus der bei 960 nm gemessenen Absorption die Konzentration von Wasser unter Berücksichtigung der Absorption durch Fett bei 930 nm und der Absorption durch Hämoglobin durch Auswertung mehrerer Absorptionen im Bereich 790-920 nm zu bestimmen.

9. Vorrichtung nach Anspruch 4, **dadurch gekennzeichnet, dass** die Datenverarbeitungseinheit (11) dazu eingerichtet ist, die Zeitreihe der wellenlängenübergreifenden Kamerasensorintensität $\overline{I}(n, t_k)$ einer Fourier-Transformation zu unterziehen und zur Erkennung des Pulssignals eine Verteilungsspitze bei einer Grundfrequenz zu suchen, die von einer oder mehreren Verteilungsspitzen von Oberwellen bei ganzzahligen Vielfachen der Grundfrequenz begleitet ist.

10. Vorrichtung nach Anspruch 9, wenn abhängig von Anspruch 5, **dadurch gekennzeichnet, dass** die Datenverarbeitungseinheit (11) dazu eingerichtet ist, nach Erkennung eines Pulssignals die Richtigkeit der Erkennung zu bestätigen, wenn in dem Signal der Kamerasensorintensität $\overline{I}_{\lambda m}(n, t_k)$ mit Am aus dem Wellenlängenbereich von 570-585 nm ein stärkeres pulsierende Signal mit der Grundfrequenz als in dem Signal der Kamerasensorintensität $\overline{I}_{\lambda m}(n, t_k)$ mit Am aus dem Wellenlängenbereich von 500-540 nm auffindbar ist.

11. Vorrichtung nach Anspruch 9 oder 10, **dadurch gekennzeichnet, dass** die Datenverarbeitungseinheit (11) dazu eingerichtet ist, in dem Fourier-Spektrum der wellenlängenübergreifenden Kamerasensorintensität $\overline{I}(n, t_k)$ ein dynamisches Bandpass-Filter für die Pulssignalerkennung anzuwenden, wobei das Zentrum des akzeptierten Frequenzbandes bei der erkannten Grundfrequenz liegt und die Breite des Frequenzbandes vorgegeben ist, wobei das dynamische Bandpass-Filter Änderungen der erkannten Grundfrequenz des Pulssignals folgt, wenn die geänderte Grundfrequenz innerhalb des akzeptierten Frequenzbandes liegt, und wobei ein hypothetisch neu erkanntes Pulssignal mit seiner Grundfrequenz verworfen wird, wenn es außerhalb des akzeptierten Frequenzbandes liegt.

12. Vorrichtung nach einem der Ansprüche 9-11, **dadurch gekennzeichnet, dass** die Datenverarbeitungseinheit (11) dazu eingerichtet ist, im Fourier-Spektrum der wellenlängenübergreifenden Kamerasensorintensität $\overline{I}(n, t_k)$ eine Verteilungsspitze mit einer Grundfrequenz, die keine begleitenden Oberwellen mit Verteilungsspitzen bei ganzzahligen Vielfachen der Grundfrequenz hat und die kleiner als die Frequenz des Pulssignals ist, der Atmung zuzuordnen und daraus die Atemfrequenz zu bestimmen.

13. Vorrichtung nach Anspruch 12, **dadurch gekennzeichnet, dass** die Datenverarbeitungseinheit (11) dazu eingerichtet ist, im Fourier-Spektrum zur Atemsignalerkennung ein dynamisches Bandpass-Filter anzuwenden, wobei das Zentrum des Frequenzbandes bei der erkannten Atemfrequenz liegt und die Breite des Frequenzbandes vorgegeben ist, wobei das dynamische Bandpass-Filter Änderungen der erkannten Atemfrequenz folgt, wenn die geänderte Atemfrequenz innerhalb des akzeptierten Frequenzbandes liegt und wobei ein hypothetisch neu erkanntes Atemsignal mit seiner neuen Atemfrequenz verworfen wird, wenn diese außerhalb des akzeptierten Frequenzbandes liegt.

14. Vorrichtung nach einem der Ansprüche 3-13, **dadurch gekennzeichnet, dass** die Datenverarbeitungseinheit dazu eingerichtet ist, die Zeitreihen $\overline{I}(i)_{\lambda m}(n, t_k)$ der Kamerasensorintensität für Emissionsmaxima Am im NIR-Wellenlängenbereich im Verhältnis zueinander und in ihren Abhängigkeiten von der Entfernung von der Beleuchtungseinheit auszuwerten, um daraus den Oxygenierungsgrad des Muskels zu bestimmen.

15. Verfahren zur kontinuierlichen und nicht invasiven Bestimmung von physiologischen Parametern eines Probanden bei körperlicher Belastung, bei dem
eine Beleuchtungseinheit (32) auf LED-Basis mit einer Mehrzahl von unterschiedlichen, nebeneinander angeordneten LED-Typen, deren Emissionsmaxima bei verschiedenen Wellenlängen $\lambda 1, A2... \lambda L$ vom sichtbaren bis in den NIR-Wellenlängenbereich liegen, in Auflage auf die Haut des Probanden an einer gewünschten Messstelle gebracht wird,
ein Fotosensor in Auflage auf die Haut des Probanden in Nachbarschaft zu der Beleuchtungseinheit gebracht wird, um von der Beleuchtungseinheit (32) ausgesendetes und durch den Körper des Probanden zu einem Austrittsort im Bereich des Fotosensor gelangendes Licht zu erfassen,
eine Datenverarbeitungseinheit (11), die zum Auslesen des Fotosensors mit diesem verbunden ist und die mit der Beleuchtungseinheit (32) in Verbindung steht, die unterschiedlichen LED-Typen jeweils einzeln in einer vorgegebenen Aktivierungssequenz zu aufeinanderfolgenden Aktivierungsstartzeitpunkten $t_k$ (k = 1, 2... M) für eine jeweils vorgegebene Aktivierungsdauer aktiviert und die Aktivierungssequenz mit einer Taktfrequenz als Folge n = 1, 2... N von Aktivierungssequenzen wiederholt, wobei die Taktfrequenz ausreichend hoch zur Auflösung des Pulses des Kreislaufs des Probanden ist, wobei in jeder Aktivierungssequenz wenigstens ein LED-Typ mit einem Emissionsmaximum unterhalb von 590 nm aktiviert wird, **dadurch gekennzeichnet, dass**
als Fotosensor ein Kamerasensor (34) auf CCD- oder CMOS-Basis mit einem zweidimensionalen Array von Sensorelementen verwendet wird,
der Kamerasensor (34) so zur Beleuchtungseinheit positioniert wird, dass er auf der Haut auf der gleichen Seite des Körperteils wie die Beleuchtungseinheit (32) und benachbart zu dieser zur Auflage kommt, um durch Transflexion durch den Körper des Probanden zum Kamerasensor (34) gelangendes Licht erfassen zu können,

die Datenverarbeitungseinheit (11) den Kamerasensor (34) in jeder Aktivierungssequenz zu den Aktivierungsstartzeitpunkten $t_k$ (k = 1, 2... M) und über die jeweilige Aktivierungsdauer ausliest und die erfassten Intensitäten der Sensorelemente über Unterbereiche von Sensorelementen zusammengefasst und der jeweiligen Aktivierungssequenz aus der Folge n = 1, 2... M von Aktivierungssequenzen und dem jeweiligen Aktivierungsstartzeitpunkt $t_k$ (k = 1, 2... M) zugeordnet als Zeitreihe aufzeichnet, wobei Unterbereiche des Kamerasensors zusammengefasst werden, indem Zeilen des Kamerasensors ausgelesen werden, die parallel zur Verbindungsachse zwischen Beleuchtungseinheit und Kamerasensor liegen und entlang derer die Entfernung von der Beleuchtungseinheit zunimmt, und parallele Zeilen zu einer einzigen gemittelten Zeile zusammengefasst werden, und die Datenverarbeitungseinrichtung dazu eingerichtet ist, die gemittelte Zeile als Funktion des Abstands von der Beleuchtungseinheit zur Muskeloximetrie auszuwerten.

**Claims**

1. Device for the continuous and non-invasive determination of physiological parameters of a test person during physical exertion, comprising

   a LED-based illumination unit (32), designed to be able to rest on the skin of the test person at a desired measuring point, including a plurality of different, juxtaposed LED types, whose emission maxima are at different wavelengths $\lambda 1$, $\lambda 2 ... \lambda L$ from visible to NIR-wavelength range, wherein the illumination unit (32) comprises at least one LED type with an emission maximum below 590 nm,

   a photo sensor, which is designed to rest on the skin of the test person in order to capture light which was emitted by the illumination unit (32) and passed through the body of the person to an exit location in the area of the photo sensor,

   a data processing unit (11), which is connected to the photo sensor for reading it out, and which is connected to the illumination unit (32) and is adapted to activate the different LED types individually in a predetermined activation sequence at successive activation start times $t_k$ (k=1, 2 ... M) for a respective predetermined activation period and to repeat the activation sequence with a clock frequency as a series n = 1, 2 ... N of activation sequences, wherein the clock frequency is sufficiently high to resolve the pulse of the test person's circulation,

   **characterized in that**

   a camera sensor (34) of CCD or CMOS type having a two-dimensional array with rows and columns of sensor elements is used as the photo sensor,

   the camera sensor (34) is arranged relative to the illumination unit (32) such that it can rest on the skin on the same side of the body part as the illumination unit and adjacent to it to detect light passing by transflection through the body of the test person to the camera sensor,

   the data processing unit (11) is set up to read out the camera sensor in each activation sequence at the activation start times $t_k$ (k=1, 2 ... M) and over the respective activation period, and to record the detected intensities of the sensor elements, combined over subregions of sensor elements and assigned to the respective activation sequence from the sequence n = 1, 2 ... N of activation sequences and the respective activation start time $t_k$ (k=1, 2 ... M) as time series, wherein sub-areas of the camera sensor are summed up by reading out rows of the camera sensor, which lie parallely to the connection axis between the illumination unit and the camera sensor and along which the distance from the illumination unit increases, and to combine parallel rows into a single averaged row, and the data processing means is arranged to analyze the averaged row as a function of the distance from the illumination unit for the purpose of muscle oximetry.

2. Device according to claim 1, **characterized in that** the data processing unit (11) is adapted to read out respective rows of the camera sensor (34), which run parallel to the connecting line between the illumination unit (32) and the camera sensor (34), and, for summarizing the sub-areas of sensor elements, to average all rows to an average row intensity

$$\bar{I}(i)_{\lambda m}(n, t_k) = \frac{1}{N_Z} \sum_{a=0}^{N_Z} I_a(i)_{\lambda m}(n, t_k)$$

where i=1, 2 ... $N_{sp}$ is the continuous column index and $N_{sp}$ is the number of columns of the camera sensor and the

index $\lambda m$ symbolizes the wavelength of the emission maximum of the respective LED type, and to record as averaged intensity $\bar{I}(i)_{\lambda m}(n, t_k)$ of the respective activation sequence n from the sequence n=1, 2 ... N of activation sequences and the respective activation start time $t_k$ (k=1, 2 ... M) as time series.

3. Device according to claim 1 or 2, **characterized in that** the data processing unit (11) is set up to sum up the intensities of the sensor elements over all rows and columns to a camera sensor intensity integrated over the camera sensor (34)

$$\bar{I}_{\lambda m} = \frac{1}{N_Z \cdot N_{Sp}} \sum_{a=0}^{N_Z} \sum_{i=0}^{N_{Sp}} I_a(i)_{\lambda m}$$

where i=1, 2 ... $N_{sp}$ symbolises the continuous column index, $N_{sp}$ the number of columns of the camera sensor, a the continuous row index, $N_z$ the number of rows of the camera sensor and the index $\lambda m$ the wavelength of the emission maximum of the respective LED type, and to record these as time series $\bar{I}_{\lambda m}(n, t_k)$ of the camera sensor intensity, assigned to the respective activation sequence n from the sequence n = 1, 2... N of activation sequences and the respective activation start time $t_k$ (k=1, 2 ... M) .

4. Device according to claim 3, **characterized in that** the data processing unit (11) is adapted to sum up the time series $\bar{I}_{\lambda m}(n, t_k)$ of the camera sensor intensity over the different wavelengths $\lambda 1, X2 ... \lambda L$ to a wavelength-spanning single time series $\bar{I}(n, t_k)$ of the camera sensor intensity and to record it.

5. Device according to one of the preceding claims, **characterized in that** the illumination unit (32) comprises at least one LED type with emission maximum in the range of 500 nm to 540 nm and an LED type with emission maximum in the range of 570 nm to 585 nm.

6. Device according to claim 5, **characterized in that** the illumination unit (32) comprises in the NIR wavelength range at least three different LED types whose emission maxima span an NIR wavelength range from 600 nm to 1100 nm.

7. Device according to claim 6, **characterized in that** the illumination unit (32) in the spanned NIR wavelength range comprises at least four different LED types with emission maxima distributed in the spanned NIR wavelength range.

8. Device according to one of the preceding claims, **characterized in that** the illumination unit (32) comprises in the NIR wavelength range a plurality of LED types with emission maxima distributed in the wavelength range 800-1100 nm, including an LED type with emission maximum at 960 nm, an LED type with emission maximum at 930 nm, and at least two LED types with emission maxima in the range of 790-920 nm, and that the data processing unit (11) is adapted to determine from the absorption measured at 960 nm the concentration of water, taking into account the absorption by fat at 930 nm and the absorption by hemoglobin by evaluating a plurality of absorptions in the range 790-920 nm.

9. Device according to claim 4, **characterized in that** the data processing unit (11) is adapted to subject the time series of the wavelength-spanning camera sensor intensity $\bar{I}(n, t_k)$ to a Fourier transformation, and to search - for detection of the pulse signal - a distribution peak at a fundamental frequency, which is accompanied by one or more distribution peaks of harmonics at integer multiples of the fundamental frequency.

10. Device according to claim 9, when dependent on claim 5, **characterized in that** the data processing unit (11) is adapted, after detection of a pulse signal, to confirm the correctness of detection, if in the signal of the camera sensor intensity $\bar{I}_{\lambda m}(n, t_k)$ with $\lambda m$ from the wavelength range of 570-585 nm a stronger pulsating signal with the fundamental frequency can be found than in the signal of the camera sensor intensity $\bar{I}_{\lambda m}(n, t_k)$) with $\lambda m$ from the wavelength range of 500-540 nm.

11. Device according to claim 9 or 10, **characterized in that** the data processing unit (11) is adapted to apply in the Fourier spectrum of the wavelength-spanning camera sensor intensity $\bar{I}(n, t_k)$ a dynamic band pass filter for the pulse signal detection, wherein the centre of the accepted frequency band is at the detected fundamental frequency and the width of the frequency band is predetermined, wherein the dynamic band pass filter follows the changes of the detected fundamental frequency of the pulse signal if the changed fundamental frequency is within the accepted frequency band, and wherein a hypothetically newly detected pulse signal with its fundamental frequency is discarded if it is outside the accepted frequency band.

12. Device according to one of claims 9-11, **characterized in that** the data processing unit (11) is set up to assign in the Fourier spectrum of the wavelength-spanning camera sensor intensity $\overline{I}(n, t_k)$ a distribution peak with a fundamental frequency, which does not have accompanying harmonics with distribution peaks at integer multiples of the fundamental frequency and which is smaller than the frequency of the pulse signal, to the respiration and to determine therefrom the respiration rate.

13. Device according to claim 12, **characterized in that** the data processing unit (11) is adapted to apply a dynamic band pass filter in the Fourier spectrum for respiratory signal detection, wherein the centre of the frequency band is at the detected respiration rate and the width of the frequency band is predetermined, wherein the dynamic band pass filter goes along with changes of the detected respiration rate if the changed respiration rate is within the accepted frequency band and wherein a hypothetically newly detected respiratory signal with its new respiration rate is discarded if it is outside the accepted frequency band.

14. Device according to one of claims 3-13, **characterized in that** the data processing unit is set up to evaluate the time series $\overline{I}(i)_{\lambda m}(n, t_k)$ of the camera sensor intensity for emission maxima Am in the NIR wavelength range in relation to each other and in their dependencies on the distance from the illumination unit to determine the degree of oxygenation of the muscle.

15. Method for the continuous and non-invasive determination of physiological parameters of a test person during physical exertion, in which
a LED-based illumination unit (32), having a plurality of different, juxtaposed LED types whose emission maxima at different wavelengths $\lambda 1$, $\lambda 2$ ... $\lambda L$ are from the visible to the NIR wavelength range, is brought in contact with the skin of the test person at a desired measuring point,
a photo sensor is placed in contact with the skin of the test person in proximity to the illumination unit in order to detect light which was emitted by the illumination unit (32) and passed through the body of the person to an exit location in the area of the photo sensor,
a data processing unit (11), which is connected to of the photo sensor for reading it out and which is in communication with the illumination unit (32), which activates each of the different LED types individually in a predetermined activation sequence at successive activation start times $t_k$ (k = 1, 2... M) for a respective predetermined activation period and repeats as a series the activation sequence at a clock frequency n = 1, 2... N of activation sequences, wherein the clock frequency is sufficiently high to resolve the pulse of the test person's blood circulation, wherein in each activation sequence at least one LED type is activated with an emission maximum below 590 nm, **characterized in that** a camera sensor (34) of CCD or CMOS type having a two-dimensional array of sensor elements is used as the photo sensor,
the camera sensor (34) is positioned relative to the illumination unit so as to rest on the skin on the same side of the body portion as the illumination unit (32) and adjacent thereto to capture light which passes by transflection through the body of the test person to the camera sensor (34),
the data processing unit (11) reads out the camera sensor (34) in each activation sequence at the activation start times tk (k = 1, 2... M) and over the respective activation period and combines the detected intensities of the sensor elements over subregions of sensor elements and assigns the respective activation sequence from the sequence n = 1, 2... M of activation sequences and the respective activation start time $t_k$ (k = 1, 2... M) and records them as a time series, wherein subareas of the camera sensor are combined by reading rows of the camera sensor which are parallel to the connection axis between the illumination unit and the camera sensor, and along which the distance from the illumination unit increases, and combines parallel lines into a single averaged row, and the data processing device is arranged to analyze the averaged row as a function of the distance from the illumination unit for the purpose of muscle oximetry.

## Revendications

1. Dispositif de détermination continue et non invasive de paramètres physiologiques d'un sujet lors d'un effort physique avec
une unité d'éclairage (32) à base de LED conçue pour reposer sur la peau du sujet en un point de mesure souhaité, avec une pluralité de différents types de LED juxtaposés, dont les maxima d'émission à différentes longueurs d'onde λ1, λ2 ... λL se situent de la gamme de longueurs d'onde visibles à la gamme de longueurs d'onde proches des infrarouges, dans lequel l'unité d'éclairage (32) comprend au moins un type de LED avec un maximum d'émission au-dessous de 590 nm,

un photo-capteur, qui est conçu pour reposer sur la peau du sujet, afin de détecter de la lumière émise par l'unité d'éclairage (32) et parvenant par le corps du sujet à un lieu de sortie dans la région du photo-capteur,

une unité de traitement de données (11), qui, pour lire le photo-capteur, est connectée à celui-ci et qui est connectée à l'unité d'éclairage (32) et est configurée pour activer les différents types de LED respectivement individuellement dans une séquence d'activation prédéterminée à des instants de début d'activation $t_k$ (k=1, 2 ... M) successifs pendant une durée d'activation respectivement prédéterminée et répéter la séquence d'activation à une fréquence d'horloge selon la suite n= 1, 2 ... N de séquences d'activation, dans lequel la fréquence d'horloge est suffisamment élevée pour la résolution du pouls de la circulation du sujet,

**caractérisé en ce que**

un capteur de caméra (34) à base CCD ou CMOS avec un réseau bidimensionnel avec des lignes et des colonnes d'éléments capteurs est utilisé comme photo-capteur,

le capteur de caméra (34) est disposé par rapport à l'unité d'éclairage (32) de sorte qu'il peut venir en appui sur la peau sur le même côté de la partie corporelle que l'unité d'éclairage et au voisinage de celle-ci, afin de pouvoir détecter de la lumière parvenant par transflexion par le corps du sujet au capteur de caméra,

l'unité de traitement de données (11) est configurée pour lire le capteur de caméra dans chaque séquence d'activation aux instants de début d'activation $t_k$ (k=1, 2 ... M) et pendant la durée d'activation respective, et pour enregistrer les intensités détectées des éléments capteurs regroupés par sous-régions d'éléments capteurs en tant que séries chronologiques associées à la séquence d'activation respective provenant de la suite n=1, 2 ... N de séquences d'activation et à l'instant de début d'activation $t_k$ (k=1, 2 ... M) respectif, dans lequel des sous-régions du capteur de caméra sont regroupées par lecture de lignes du capteur de caméra, qui se situent parallèlement à l'axe de connexion entre l'unité d'éclairage et le capteur de caméra et le long desquelles la distance par rapport à l'unité d'éclairage augmente, et des lignes parallèles sont regroupées en une seule ligne moyenne, et l'appareil de traitement de données est configuré pour évaluer la ligne moyenne en fonction de l'écart par rapport à l'unité d'éclairage aux fins d'oxymétrie des muscles.

2. Dispositif selon la revendication 1, **caractérisé en ce que** l'unité de traitement de données (11) est configurée pour lire respectivement des lignes du capteur de caméra (34), qui s'étendent parallèlement à la ligne de connexion entre l'unité d'éclairage (32) et le capteur de caméra (34), et, pour le regroupement de sous-régions d'éléments capteurs, pour calculer la moyenne de toutes les lignes pour obtenir une intensité de ligne moyenne

$$\bar{I}(i)_{\lambda m}(n, t_k) = \frac{1}{N_Z} \sum_{a=0}^{N_Z} I_a(i)_{\lambda m}(n, t_k)$$

dans lequel i=1,2 ... $N_{sp}$ est l'indice de colonne continu et $N_{sp}$ le nombre de colonnes du capteur de caméra et l'indice λm symbolise la longueur d'onde du maximum d'émission du type de LED respectif, et pour enregistrer en tant qu'intensité moyenne $\bar{I}(i)_{\lambda m}(n, t_k)$ en tant que séries chronologiques associées à la séquence d'activation n respective provenant de la suite n=1, 2 ... N de séquences d'activation et à l'instant de début d'activation $t_k$ (k=1, 2 ... M) respectif.

3. Dispositif selon la revendication 1 ou 2, **caractérisé en ce que** l'unité de traitement de données (11) est configurée pour regrouper les intensités des éléments capteurs sur toutes les lignes et colonnes en une intensité de capteur de caméra

$$\bar{I}_{\lambda m} = \frac{1}{N_Z \cdot N_{Sp}} \sum_{a=0}^{N_Z} \sum_{i=0}^{N_{Sp}} I_a(i)_{\lambda m}$$

intégrée sur le capteur de caméra (34), dans lequel i=1, 2 ... $N_{sp}$ symbolise l'indice de colonne continu, $N_{sp}$ le nombre de colonnes du capteur de caméra, a l'indice de ligne continu, $N_z$ le nombre de lignes du capteur de caméra et l'indice λm la longueur d'onde du maximum d'émission du type de LED respectif, et pour enregistrer celles-ci en tant que séries chronologiques $\overline{I}_{\lambda m}(n, t_k)$ de l'intensité de capteur de caméra associées à la séquence d'activation n respective provenant de la suite n=1, 2 ... N de séquences d'activation et à l'instant de début d'activation $t_k$ (k=1, 2 ... M) respectif.

4. Dispositif selon la revendication 3, **caractérisé en ce que** l'unité de traitement de données (11) est configurée pour regrouper et enregistrer les séries chronologiques $\overline{I}_{\lambda m}(n, t_k)$ de l'intensité de capteur de caméra des différentes longueurs d'onde $\lambda 1$, $\lambda 2$ ... $\lambda L$ en une série chronologique individuelle $\overline{I}(n, t_k)$ couvrant les longueurs d'onde, de l'intensité de capteur de caméra.

5. Dispositif selon l'une quelconque des revendications précédentes, **caractérisé en ce que** l'unité d'éclairage (32) comprend au moins un type de LED avec un maximum d'émission dans la gamme de 500 nm à 540 nm et un type de LED avec un maximum d'émission dans la gamme de 570 nm à 585 nm.

6. Dispositif selon la revendication 5, **caractérisé en ce que** l'unité d'éclairage (32) comprend dans la gamme de longueurs d'onde proches des infrarouges au moins trois différents types de LED, dont les maxima d'émission couvrent une gamme de longueurs d'ondes proches des infrarouges de 600 nm à 1 100 nm.

7. Dispositif selon la revendication 6, **caractérisé en ce que** l'unité d'éclairage (32) comprend dans la gamme de longueurs d'onde proches des infrarouges couverte au moins quatre différents types de LED avec des maxima d'émission distribués dans la gamme de longueurs d'onde proches des infrarouges couverte.

8. Dispositif selon l'une quelconque des revendications précédentes, **caractérisé en ce que** l'unité d'éclairage (32) présente dans la gamme de longueurs d'onde proches des infrarouges une pluralité de types de LED avec des maxima d'émission distribués dans la gamme de longueurs d'onde de 800-1100 nm, y compris un type de LED avec un maximum d'émission à 960 nm, un type de LED avec un maximum d'émission à 930 nm et au moins deux types de LED avec des maxima d'émission dans la gamme de 790-920 nm, et **en ce que** l'unité de traitement de données (11) est configurée pour déterminer à partir de l'absorption mesurée à 960 nm la concentration d'eau avec prise en compte de l'absorption par le gras à 930 nm et de l'absorption par l'hémoglobine par évaluation de plusieurs absorptions dans la gamme de 790-920 nm.

9. Dispositif selon la revendication 4, **caractérisé en ce que** l'unité de traitement de données (11) est configurée pour soumettre la série chronologique de l'intensité de capteur de caméra $\overline{I}(n, t_k)$ couvrant les longueurs d'onde à une transformée de Fourier et, pour détecter le signal de pouls, pour rechercher un pic de distribution à une fréquence fondamentale qui est accompagnée d'un ou de plusieurs pics de distribution d'harmoniques à des multiples entiers de la fréquence fondamentale.

10. Dispositif selon la revendication 9, lorsqu'elle dépend de la revendication 5, **caractérisé en ce que** l'unité de traitement de données (11) est configurée pour confirmer, après détection d'un signal de pouls, l'exactitude de la détection, si dans le signal de l'intensité de capteur de caméra $\overline{I}_{\lambda m}(n, t_k)$ avec $\lambda m$ provenant de la gamme de longueurs d'onde de 570-585 nm, un signal pulsatoire avec la fréquence fondamentale plus fort que dans le signal de l'intensité de capteur de caméra $\overline{I}_{\lambda m}(n, t_k)$ avec $\lambda m$ provenant de la gamme de longueurs d'onde 500-540 nm peut être localisé.

11. Dispositif selon la revendication 9 ou 10, **caractérisé en ce que** l'unité de traitement de données (11) est configurée pour appliquer dans le spectre de Fourier de l'intensité de capteur de caméra $\overline{I}(n, t_k)$ couvrant les longueurs d'onde un filtre passe-bas dynamique pour la détection de signal de pouls, dans lequel le centre de la bande de fréquences acceptée se situe à la fréquence fondamentale détectée et la largeur de la bande de fréquences est prédéterminée, dans lequel le filtre passe-bas dynamique suit des modifications de la fréquence fondamentale détectée du signal de pouls, si la fréquence fondamentale modifiée se situe à l'intérieur de la bande de fréquences acceptée, et dans lequel un signal de pouls hypothétiquement à nouveau détecté avec sa fréquence fondamentale est rejeté, s'il se situe à l'extérieur de la bande de fréquences acceptée.

12. Dispositif selon l'une quelconque des revendications 9 à 11, **caractérisé en ce que** l'unité de traitement de données (11) est configurée pour associer dans le spectre de Fourier de l'intensité de capteur de caméra $\overline{I}(n, t_k)$ couvrant les longueurs d'onde un pic de distribution avec une fréquence fondamentale, qui ne présente pas d'harmoniques concomitants avec des pics de distribution à des multiples entiers de la fréquence fondamentale et qui est inférieure à la fréquence du signal de pouls, à la respiration et pour déterminer à partir de cela la fréquence respiratoire.

13. Dispositif selon la revendication 12, **caractérisé en ce que** l'unité de traitement de données (11) est configurée pour appliquer un filtre passe-bas dynamique dans le spectre de Fourier pour la détection du signal respiratoire, dans lequel le centre de la bande de fréquences se situe à la fréquence respiratoire détectée et la largeur de la bande de fréquences est prédéterminée, dans lequel le filtre passe-bas dynamique suit des modifications de la fréquence respiratoire détectée, si la fréquence respiratoire modifiée se situe à l'intérieur de la bande de fréquences acceptée et dans lequel un signal de respiration hypothétiquement à nouveau détecté avec sa nouvelle fréquence respiratoire est rejeté si celle-ci se situe à l'extérieur de la bande de fréquences acceptée.

14. Dispositif selon l'une quelconque des revendications 3 à 13, **caractérisé en ce que** l'unité de traitement de données est configurée pour évaluer les séries chronologiques $\overline{I}(i)_{\lambda m}(n, t_k)$ de l'intensité de capteur de caméra pour des maxima d'émission λm dans la gamme de longueurs d'onde proches des infrarouges les unes par rapport aux autres et dans leurs dépendances de la distance par rapport à l'unité d'éclairage, afin de déterminer à partir de cela le degré d'oxygénation du muscle.

15. Procédé pour la détermination continue et non invasive de paramètres physiologiques d'un sujet lors d'un effort physique, selon lequel
une unité d'éclairage (32) à base de LED avec une pluralité de différents types de LED juxtaposées, dont les maxima d'émission à différentes longueurs d'onde λ1, λ2 ... λL se situent de la gamme de longueurs d'onde visibles à la gamme de longueurs d'onde proches des infrarouges, est placée en appui sur la peau du sujet en un point de mesure souhaité,
un photo-capteur est placé en appui sur la peau du sujet au voisinage de l'unité d'éclairage, afin de détecter de la lumière émise par l'unité d'éclairage (32) et parvenant par le corps du sujet à un lieu de sortie dans la région du photo-capteur,
une unité de traitement de données (11), qui, pour lire le photo-capteur, est connectée à celui-ci et qui est connectée à l'unité d'éclairage (32), qui active les différents types de LED respectivement individuellement dans une séquence d'activation prédéterminée à des instants de début d'activation $t_k$ (k = 1, 2 ... M) successifs pendant une durée d'activation respectivement prédéterminée et répète la séquence d'activation à une fréquence d'horloge selon la suite n = 1, 2 ... N de séquences d'activation, dans lequel la fréquence d'horloge est suffisamment élevée pour la résolution du pouls de la circulation du sujet, dans lequel dans chaque séquence d'activation, au moins un type de LED avec un maximum d'émission au-dessous de 590 nm est activé, **caractérisé en ce que**
un capteur de caméra (34) à base CCD ou CMOS avec un réseau bidimensionnel d'éléments capteurs est utilisé comme photo-capteur,
le capteur de caméra (34) est positionné par rapport à l'unité d'éclairage de sorte qu'il vient en appui sur la peau sur le même côté de la partie corporelle que l'unité d'éclairage (32) et au voisinage de celle-ci, afin de pouvoir détecter de la lumière parvenant par transflexion par le corps du sujet au capteur de caméra (34),
l'unité de traitement de données (11) lit le capteur de caméra (34) dans chaque séquence d'activation aux instants de début d'activation $t_k$ (k=1, 2 ... M) et pendant la durée d'activation respective et enregistre les intensités détectées des éléments capteurs regroupés par sous-régions d'éléments capteurs en tant que séries chronologiques associées à la séquence d'activation respective provenant de la suite n = 1, 2 ... N de séquences d'activation et à l'instant de début d'activation $t_k$ (k=1, 2 ... M) respectif, dans lequel des sous-régions du capteur de caméra sont regroupées, par lecture de lignes du capteur de caméra, qui se situent parallèlement à l'axe de connexion entre l'unité d'éclairage et le capteur de caméra et le long desquelles la distance par rapport à l'unité d'éclairage augmente, et des lignes parallèles sont regroupées en une seule ligne moyenne, et l'appareil de traitement de données est configuré pour évaluer la ligne moyenne en fonction de l'écart entre l'unité d'éclairage et l'oxymétrie des muscles.

Fig. 1

EP 3 399 914 B1

EP 3 399 914 B1

Fig. 2

Fig. 3

EP 3 399 914 B1

| 1 | 2 | 3 | 4 |
|---|---|---|---|
| 660nm | 720nm | 805nm | 525nm |

gem.
Kath.

| 8 | 7 | 6 | 5 |
|---|---|---|---|
| 760nm | | 910nm | 575nm |

Fig. 4

EP 3 399 914 B1

Fig. 5

Fig. 6

arterielles Blut

Absorption

Mikrozirkulation/ Gewebe

venöses Blut

arterielle und
Puls-Parameter:
**art. Versorgung, HR, PI, HRV**

Gewebe- bzw.
Muskel-Parameter:
**$S_mO_2$, THI**

durch venöse Verschiebung:
**RR, Bewegungsartefakte**

Fig. 7

Fig. 8

23 überlagertes Zeitsignal $\bar{I}[n]$

22 Atmung

21 Puls

20 Bewegung

t

Fig. 9

EP 3 399 914 B1

Fig. 10

EP 3 399 914 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20120253153 A1
- US 20150282724 A1
- US 20150282746 A1
- WO 2014139830 A1
- US 2015297133 A1
- WO 2011091280 A2
- US 2008097173 A1

- WO 2010053617 A2
- WO 2004097365 A2
- WO 2010093865 A1
- WO 2013158459 A1
- US 2014016132 A1
- US 20150196238 A1
- US 201501962381 A

### In der Beschreibung aufgeführte Nicht-Patentliteratur

- **BABS R. SOLLER et al.** Noninvasive determination of exercise-induced hydrogen ion threshold through direct optical measurement. *J Appl Physiol,* 2008, vol. 104, 837-844

- **G. ELLERBY et al.** Validation of a spectroscopic sensor for the continuous, noninvasive measurement of muscle oxygen saturation and pH. *Physiol. Meas.,* 2013, vol. 34, 859-871
- **MANNHEIMER PH.D.** The light-tissue interaction of pulse oximetry. *Anesth. Analg.,* Dezember 2007, vol. 105 (6), 10-7